# EUROPEAN PATENT SPECIFICATION

(11) **EP 4 316 492 B1**
(45) Date of publication and mention of the grant of the patent: **03.12.2025**
(21) Application number: 22778940.1
(22) Date of filing: 29.03.2022
(51) Int. Cl.: A61K 31/5517, A61K 39/395, A61P 35/00, A61P 35/02, A61P 35/04, A61K 45/06, A61K 39/00, C07K 16/28

(54) **COMBINATION OF A MULTIKINASE INHIBITOR WITH A PD-1/PD-L1 ANTIBODY AND USES THEREOF**
KOMBINATION EINES MULTIKINASEINHIBITORS MIT EINEM PD-1/PD-L1-ANTIKÖRPER UND VERWENDUNGEN DAVON
COMBINAISON D'UN INHIBITEUR DE MULTIKINASE AVEC UN ANTICORPS PD-1/PD-L1 ET DES UTILISATIONS DE CELUI-CI

(30) Priority: 29.03.2021 CN 202110333657
(43) Date of publication of application: 07.02.2024
(73) Proprietor: Transthera Sciences (Nanjing), Inc., Jiangbei New Area Nanjing Jiangsu 210032 (CN)
(72) Inventor: PENG, Peng, Nanjing, Jiangsu 210032 (CN); QIANG, Xiaoyan, Nanjing, Jiangsu 210032 (CN); WU, Frank, Nanjing, Jiangsu 210032 (CN)
(74) Representative: Grünecker Patent- und Rechtsanwälte PartG mbB
(86) International application number: PCT/CN2022/083685
(87) International publication number: WO 2022/206758

(56) References cited:
- WO-A1-2018/108079
- WO-A1-2019/161000
- CN-A- 102 603 743
- CN-A- 109 675 040
- CN-A- 113 620 949
- PENG PENG ET AL: "Abstract 5313: TT-00420, a novel multiple kinase inhibitor to treat TNBC | Cancer Research | American Association for Cancer Research", vol. 80, no. 16_Supplement, 15 August 2020 (2020-08-15), San Diego, CA . Philadelphia (PA, pages 5313 - 5313, XP093231846, ISSN: 0008-5472, Retrieved from the Internet <URL:https://aacrjournals.org/cancerres/article/80/16_Supplement/5313/643914/Abstract-5313-TT-00420-a-novel-multiple-kinase> DOI: 10.1158/1538-7445.AM2020-5313
- PENG PENG ET AL: "Tinengotinib (TT-00420), a Novel Spectrum-Selective Small-Molecule Kinase Inhibitor, Is Highly Active Against Triple-Negative Breast Cancer", vol. 22, no. 2, 12 October 2022 (2022-10-12), US, pages 205 - 214, XP093231882, ISSN: 1535-7163, Retrieved from the Internet <URL:https://aacrjournals.org/mct/article-pdf/22/2/205/3266241/205.pdf> DOI: 10.1158/1535-7163.MCT-22-0012

## Description

### TECHNICAL FIELD

The present invention belongs to the technical field of pharmaceuticals, and particularly relates to combined use of a multi-kinase inhibitor and a programmed cell death protein 1/programmed cell death protein 1 ligand 1 (PD-1/PD-L1) antibody.

### BACKGROUND

Normal cell division is essential for the health of the body and the survival of cellular organs. During this process, the intracellular material is completely recombined, and two identical chromosome copies are separated into two daughter cells by a bipolar spindle. When an error occurs in the mitosis process, chromosomal number in the cell will be abnormal, which may lead to cell death or promote the development of normal cells to tumor cells. The mitosis process mainly depends on three mechanisms: ① protein localization; ② proteolysis; and ③ phosphorylation. During the processes, some serine/threonine kinases, also known as mitotic kinases, are involved.

Aurora kinase is one kind of the mitotic kinases and was discovered in 1995. The expression of Aurora kinase was first observed in human tumor tissue in 1998. It has now become a target of concern for anti-tumor research. The Aurora kinase family includes three highly homologous kinases: Aurora A, Aurora B, and Aurora C. Among them, Aurora A and Aurora B have reached detectable levels so far.

Aurora A has now been demonstrated to be an oncogene, whose overexpression blocks the correct assembly of mitotic checkpoint complexes, resulting in genetic instability and tumor formation. Aurora B is an important kinase that regulates normal cell mitosis. Overexpression of Aurora B is widespread in tumors. Tumor cells become more sensitive when Aurora B is inhibited. In view of the key roles of Aurora A and Aurora B in the process of cell mitosis, the research and development of anti-tumor drugs targeting Aurora kinases have attracted more and more attention. In addition, Aurora kinases are ineffective against non-proliferating cells since they are expressed and activated in mitosis. Therefore, Aurora kinase inhibitors belong to targeted anti-tumor drugs, which will have greater advantages over other non-specific cytotoxic drugs. Compositions comprising an Aurora kinase inhibitor and an immune checkpoint inhibitor to treat cancer are described in the art, e.g. in CN109675040A.

In addition to being associated with overexpression of mitotic kinases, tumor growth and migration also depend on the formation of a large number of new blood vessels, in which VEGF/VEGFR (vascular endothelial growth factor/vascular endothelial growth factor receptor) pathway plays a key role in tumor neovascularization. Among them, VEGFR is a type of tyrosine kinase transmembrane glycoprotein consisting of an extracellular region composed of 7 Ig-like domains, one transmembrane domain, and a cytoplasmic tyrosine kinase domain. There are three subtypes of VEGFR, which are VEGFR1, VEGFR2, and VEGFR3. The conformation of VEGFR changes after bonding to VEGF, which leads to dimerization of the receptor, autophosphorylation of the tyrosine site in the intracellular segment, and activation of downstream signal transduction pathway. Among them, VEGFR2 (KDR) is mainly distributed in vascular endothelial cells and hematopoietic stem cells. VEGFR2 (KDR) is closely related to hematopoietic system dysfunction before malignant proliferative lesions, such as thrombocythemia, primary thrombocythemia, myelofibrosis (MF), chronic idiopathic myelofibrosis (IMF), polycythemia (PV), pre-cancerous myelodysplastic syndrome, and hematological malignancies. Among them, hematological malignancies include, but are not limited to, leukemia (non-Hodgkin's lymphoma), Hodgkin's disease (also known as Hodgkin's lymphoma), and myeloma, for example, acute lymphoblastic leukemia (ALL), acute myeloid leukemia (AML), acute promyelocytic leukemia (APL), chronic lymphocytic leukemia (CLL), chronic myeloid leukemia (CML), chronic neutrophilic leukemia (CNL), etc.

Results from recent studies demonstrate that inhibition of VEGFR pathways can improve the microenvironment inside the tumor, partially removing immunosuppression. E7080 (Lenvatinib) is a small molecule tyrosine kinase inhibitor that inhibits the kinase activity of the vascular endothelial growth factor (VEGF) receptors VEGFR1 (FLT1), VEGFR2 (KDR), and VEGFR3 (FLT4).

A set of clinical data published by the American Society of Clinical Oncology (ASCO) in 2017 is attractive, in which the PD-1 antibody is combined with the targeted drug E7080 for targeting endometrial cancer, with a control rate of 96%. In addition, based on these clinical data, Merck has signed a partnership with Eisai, the research and development company of E7080, for up to $5.755 billion for clinical development in combination with the PD-1 antibody. The clinical research on the combination therapy of PD-1 antibody and E7080 for other tumor types is expanded, including non-small cell lung cancer, renal cell carcinoma, uterine adenocarcinoma, bladder cancer, head and neck squamous carcinoma, and melanoma.

CSF1R is a receptor tyrosine kinase whose ligand is CSF1, and primarily regulates the distribution of tumor-associated macrophages (TAMs) in tumors. TAM is a collective term for a class of macrophages that are locally abundant in tumors, accounting for 50% of tumor tissue. TAMs can be classified as M1 and M2 type macrophages. Among them, M2 type macrophages can generate inhibitory effects on proliferation and activation of T cells, thereby generating an immunosuppressive tumor microenvironment; M1 type macrophages have opposite effects compared to M2 type macrophages and can produce killing effects on tumor cells. TAMs in the tumor microenvironment are predominantly of M2 type. It is found by study that blocking CSF1/CSFIR signals can lead to preferential depletion of M2 type tumor-associated macrophages, whereas M1 type tumor-associated macrophages are less affected, and can promote M2 type macrophages to transform into M1 type macrophages. CSF1R inhibitors are therefore developed to be useful in regulating the immunosuppressive tumor microenvironment. In addition, it is reported in the literature that the drug resistance of PD-1 is related to the expression of TAMs within the tumor microenvironment, and therefore the development of CSF1R inhibitors can solve the problem of PD-1 drug resistance caused by TAMs. Therefore, the combination of the CSF1R inhibitor and the PD-1 antibody has become a hot topic for the study of tumor immunotherapy.

Janus kinase (JAK)/signal transducer and activator of transcription (STAT) signaling pathway is an important signal transduction pathway involved in the regulation of cell differentiation, proliferation, apoptosis, neovascularization, and immunity by various extracellular signals (interferons, cytokines, growth factors, hormones, polypeptides, etc.). In the process of tumorigenesis, different pathological conditions can cause abnormal activation of JAK/STAT pathway, thereby promoting the occurrence, development, immune escape, and metastasis of tumors.

Studies show that interferon-activated upregulation of indoleamine 2,3-dioxygenase (IDO) is mediated by JAK/STAT. The regulation of IDO on the immune system mainly includes the following aspects: (1) The high expression of IDO can lead to the local depletion of tryptophan in cells. Because T cells are particularly sensitive to the depletion of tryptophan, when the concentration of tryptophan decreases, the proliferation of T cells will be arrested in G1 phase. (2) IDO-dependent tryptophan degradation leads to the increase of kynurenine level and induces oxygen radical-mediated T cell apoptosis. (3) Upregulation of IDO expression in dendritic cells strengthens local regulatory T cell (Treg)-mediated immunosuppression by degrading local tryptophan, and promotes the peripheral immune tolerance of the body to tumor-specific antigen. Therefore, the JAK/STAT pathway activated abnormally in tumor cells leads to a significant increase in IDO expression, which is one of the important mechanisms by which tumor cells escape the immune system. At present, overexpression of IDO has been found in various tumor cells such as pancreatic cancer cells, breast cancer cells, and gastric cancer cells.

The above results from studies indicate that multi-target inhibitors, in addition to being effective in monotherapy, also have broad prospects in combination therapy.

### SUMMARY

A compound of formula (I) of the present invention is a multi-target compound for inhibiting cell cycle and regulating tumor microenvironment, has good inhibitory activity on VEGFR1 (FLT1), VEGFR2 (KDR), VEGFR3 (FLT4), CKIT, PDGFR, CSF1R, JAK, etc., and also has better tumor therapeutic effect when used in combination with immune checkpoint inhibitors such as PD-1 antibody.

The present invention provides a pharmaceutical composition of a multi-kinase inhibitor compound shown in formula (I) or a pharmaceutically acceptable salt, a stereoisomer or a crystal form thereof and a programmed cell death protein 1/programmed cell death protein 1 ligand 1 (PD-1/PD-L1) antibody, 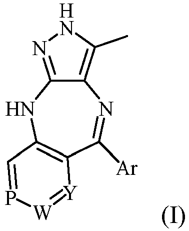 wherein,
Ar is selected from phenyl and 5-6 membered heteroaryl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, oxa-C₅₋₈ cycloalkyloxy, halogenated C₁₋₄ alkoxy, C₃₋₆ cycloalkylamino, C₁₋₄ alkylsulfonyl, C₃₋₆ cycloalkylsulfonyl, C₁₋₄ alkylcarbonyl, C₃₋₆ cycloalkylcarbonyl, -(CH₂)ₙ-C₃₋₁₀ cycloalkyl, -(CH₂)ₙ-(5-11) membered heterocyclyl, and - (CH₂)ₙ-(5-10) membered heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

In another embodiment, the present invention provides a pharmaceutical composition comprising a multi-kinase inhibitor compound shown in formula (I) or a pharmaceutically acceptable salt, a stereoisomer or a crystal form thereof and a programmed cell death protein 1/programmed cell death protein 1 ligand 1 (PD-1/PD-L1) antibody, and a pharmaceutically acceptable carrier, 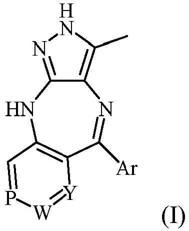 wherein,
Ar is selected from phenyl and 5-6 membered heteroaryl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, oxa-C₅₋₈ cycloalkyloxy, halogenated C₁₋₄ alkoxy, C₃₋₆ cycloalkylamino, C₁₋₄ alkylsulfonyl, C₃₋₆ cycloalkylsulfonyl, C₁₋₄ alkylcarbonyl, C₃₋₆ cycloalkylcarbonyl, -(CH₂)ₙ-C₃₋₁₀ cycloalkyl, -(CH₂)ₙ-(5-11) membered heterocyclyl, and - (CH₂)ₙ-(5-10) membered heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

In another embodiment, the present invention provides a pharmaceutical composition consisting of a multi-kinase inhibitor compound shown in formula (I) or a pharmaceutically acceptable salt, a stereoisomer or a crystal form thereof and a programmed cell death protein 1/programmed cell death protein 1 ligand 1 (PD-1/PD-L1) antibody, and a pharmaceutically acceptable carrier, 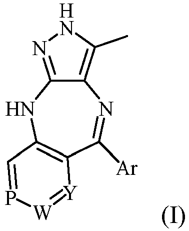 wherein,
Ar is selected from phenyl and 5-6 membered heteroaryl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, oxa-C₅₋₈ cycloalkyloxy, halogenated C₁₋₄ alkoxy, C₃₋₆ cycloalkylamino, C₁₋₄ alkylsulfonyl, C₃₋₆ cycloalkylsulfonyl, C₁₋₄ alkylcarbonyl, C₃₋₆ cycloalkylcarbonyl, -(CH₂)ₙ-C₃₋₁₀ cycloalkyl, -(CH₂)ₙ-(5-11) membered heterocyclyl, and - (CH₂)ₙ-(5-10) membered heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

In another embodiment, in formula (I):
Ar is selected from phenyl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, halogenated C₁₋₄ alkoxy, C₃₋₆ cycloalkylamino, C₁₋₄ alkylsulfonyl, C₁₋₄ alkylcarbonyl, C₃₋₆ cycloalkyl-carbonyl, -(CH₂)ₙ-C₃₋₆ cycloalkyl, -(CH₂)ₙ-(5-6) membered monocyclic heterocyclyl, -(CH₂)ₙ-(7-11) membered fused heterocyclyl, - (CH₂)ₙ-(5-6) membered monocyclic heteroaryl, and -(CH₂)ₙ-(8-10) membered fused heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

In another embodiment, formula (I) is a compound described in Table 1.

In another embodiment, formula (I) is compound 1 described in Table 1.

The present invention also provides a kit for treating a cancer, comprising (a) the multi-kinase inhibitor compound shown in formula (I) or the pharmaceutically acceptable salt, the stereoisomer or the crystal form thereof described above, (b) a programmed cell death protein 1/programmed cell death protein 1 ligand 1 (PD-1/PD-L1) antibody, and (c) a package insert.

In another embodiment, in formula (I):
Ar is selected from phenyl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, halogenated C₁₋₄ alkoxy, C₃₋₆ cycloalkylamino, C₁₋₄ alkylsulfonyl, C₁₋₄ alkylcarbonyl, C₃₋₆ cycloalkyl-carbonyl, -(CH₂)ₙ-C₃₋₆ cycloalkyl, -(CH₂)ₙ-(5-6) membered monocyclic heterocyclyl, -(CH₂)ₙ-(7-11) membered fused heterocyclyl, - (CH₂)ₙ-(5-6) membered monocyclic heteroaryl, and -(CH₂)ₙ-(8-10) membered fused heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

In another embodiment, formula (I) is a compound described in Table 1.

In another embodiment, formula (I) is compound 1 described in Table 1.

**Table 1**

| Compound | Structure | Compound | Structure |
|---|---|---|---|
| 1 | 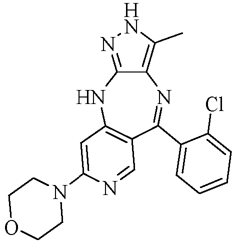 | 2 | 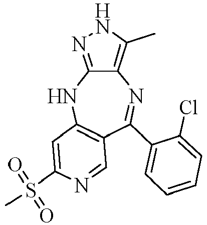 |
| 3 | 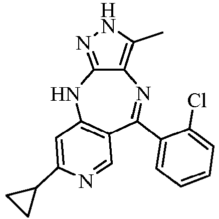 | 4 | 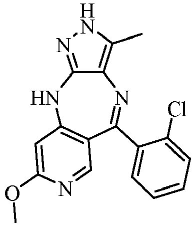 |
| 5 | 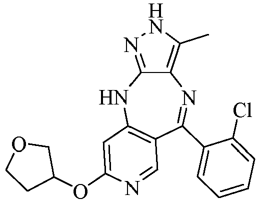 | 6 | 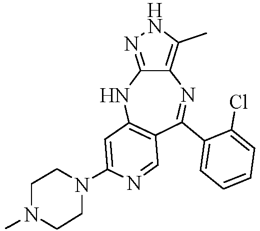 |
| 7 | 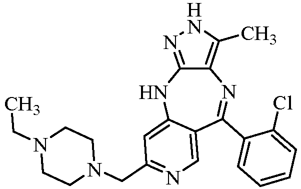 | 8 | 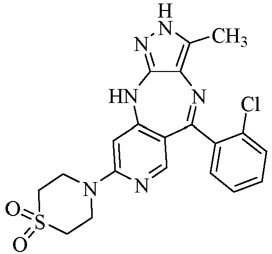 |
| 9 | 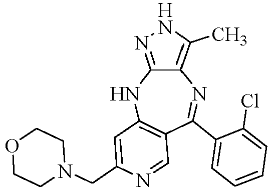 | 10 | 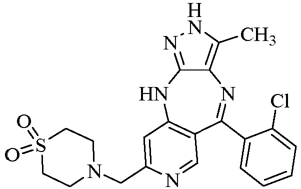 |
| 11 | 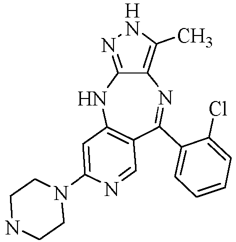 | 12 | 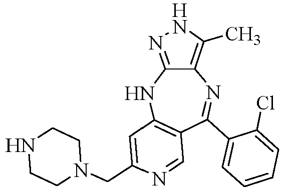 |
| 13 | 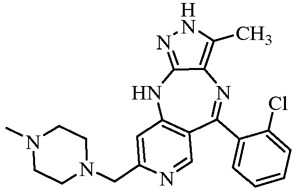 | 14 | 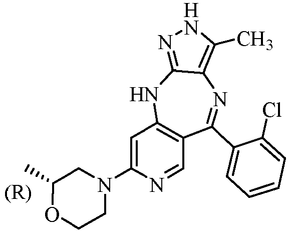 |
| 15 | 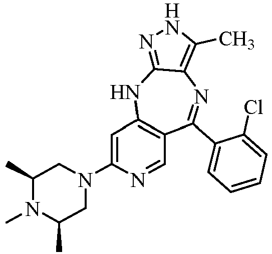 | 16 | 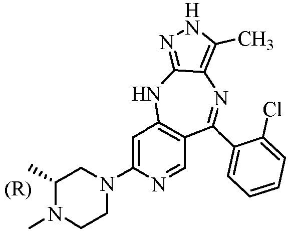 |
| 17 | 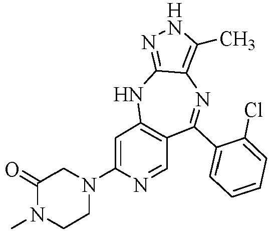 | 18 | 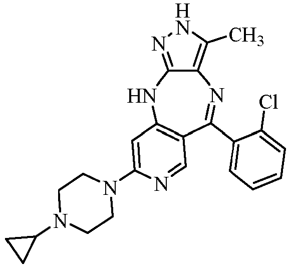 |
| 19 | 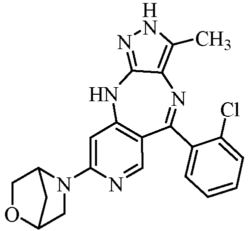 | 20 | 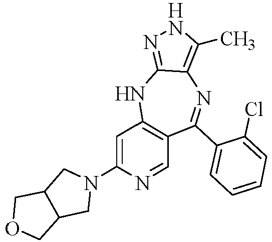 |
| 21 | 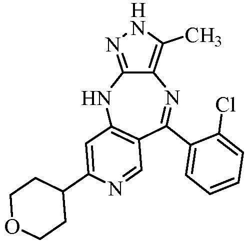 | 22 | 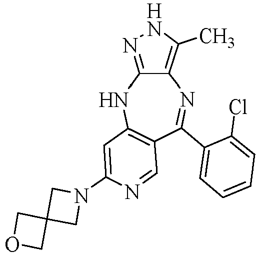 |
| 23 | 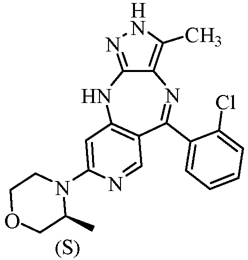 | 24 | 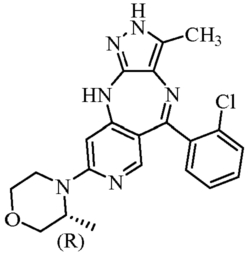 |
| 25 | 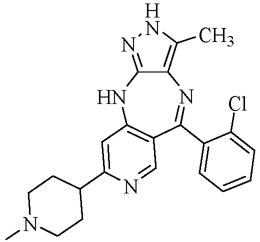 | | |

In some embodiments, the pharmaceutical composition or the kit of the present invention may further comprise an additional anti-cancer agent or immunosuppressant.

In some embodiments, the additional anti-cancer agent is selected from a chemotherapeutic agent or a radiotherapeutic agent, and the chemotherapeutic agent is selected from one of or a combination of more of chlorambucil, ifosfamide, doxorubicin, mesalazine, thalidomide, lenalidomide, temsirolimus, everolimus, fludarabine, paclitaxel, docetaxel, ofatumumab, rituximab, ibritumomab tiuxetan, tositumomab, dexamethasone, prednisone, bortezomib, pentostatin, and endostatin.

In some embodiments, the additional immunosuppressant is selected from one of or a combination of more of PD-1, PD-L1, CTAL-4, PD-L2, LAG3, TIM3, 2B4, A2Ar, B7H1, B7H3, B7H4, BTLA, CD2, CD27, CD28, CD30, CD40, CN70, CD80, CD86, CD137, CD160, CD226, CD276, DR3, GAL9, GIRT, HAVCR2, HVEM, ICOS, KIR, LAIR1, LIGHT, MARCO, PS, OX40, SLAM, TIGHT, VISTA, and VTCN1 antibodies.

The present invention also provides use of the pharmaceutical composition and the kit of the multi-kinase inhibitor compound shown in formula (I) or the pharmaceutically acceptable salt, the stereoisomer or the crystal form thereof and the programmed cell death protein 1/programmed cell death protein 1 ligand 1 (PD-1/PD-L1) antibody in the manufacture of a medicament for preventing and/or treating a cancer mediated by multi-kinase abnormalities.

The present invention also provides the pharmaceutical composition or the kit of the multi-kinase inhibitor compound shown in formula (I) or the pharmaceutically acceptable salt, the stereoisomer or the crystal form thereof and the programmed cell death protein 1/programmed cell death protein 1 ligand 1 (PD-1/PD-L1) antibody for use in the prevention and/or treatment of a cancer mediated by multi-kinase abnormalities.

The present invention also provides a method for preventing and/or treating a cancer mediated by multi-kinase abnormalities, which comprises administering to a subject the pharmaceutical composition or the kit of the multi-kinase inhibitor compound shown in formula (I) or the pharmaceutically acceptable salt, the stereoisomer or the crystal form thereof and the programmed cell death protein 1/programmed cell death protein 1 ligand 1 (PD-1/PD-L1) antibody.

In some embodiments, the present invention provides a multi-kinase inhibitor compound shown in formula (I) or a pharmaceutically acceptable salt, a stereoisomer or a crystal form thereof for preventing and/or treating a cancer, wherein the multi-kinase inhibitor compound shown in formula (I) or the pharmaceutically acceptable salt, the stereoisomer or the crystal form thereof is administered to an individual in combination with a programmed cell death protein 1/programmed cell death protein 1 ligand 1 (PD-1/PD-L1) antibody.

In some embodiments, the present invention provides a programmed cell death protein 1/programmed cell death protein 1 ligand 1 (PD-1/PD-L1) antibody for preventing and/or treating a cancer, wherein the programmed cell death protein 1/programmed cell death protein 1 ligand 1 (PD-1/PD-L1) antibody is administered to an individual in combination with a multi-kinase inhibitor compound shown in formula (I) or a pharmaceutically acceptable salt, a stereoisomer or a crystal form thereof.

The present invention also provides use of the multi-kinase inhibitor compound shown in formula (I) or the pharmaceutically acceptable salt, the stereoisomer or the crystal form thereof in combination with the programmed cell death protein 1/programmed cell death protein 1 ligand 1 (PD-1/PD-L1) antibody in preventing and/or treating a cancer.

In some embodiments, the present invention provides a dose of the programmed cell death protein 1/programmed cell death protein 1 ligand 1 (PD-1/PD-L1) antibody, and the multi-kinase inhibitor compound shown in formula (I) or the pharmaceutically acceptable salt, the stereoisomer or the crystal form thereof effective to combination therapy of a cancer.

In some embodiments, the frequency of administration of the PD-1 antibody or PD-L1 antibody will vary with the type and severity of the disease, for example, the frequency of administration of the PD-1 antibody or PD-L1 antibody is once a week, once every two weeks, once every three weeks, once every four weeks, once every five weeks, once every six weeks, once every seven weeks, or once every eight weeks.

In another embodiment, the cancer includes lung cancer, squamous cell carcinoma, bladder cancer, gastric cancer, ovarian cancer, peritoneal cancer, breast cancer, head and neck cancer, endometrial cancer, corpus uteri cancer, rectal cancer, liver cancer, cholangiocarcinoma, gallbladder carcinoma, pancreatic cancer, renal cancer, renal pelvis cancer, esophageal cancer, esophageal adenocarcinoma, glioma, thyroid cancer, female reproductive system cancer, neurofibromatosis, bone cancer, skin cancer, brain cancer, colon cancer, testicular cancer, oral cancer, pharyngeal cancer, hematological cancer, villous adenoma of large intestine, melanoma, cytoma, and sarcoma.

In some embodiments, the cancer is lung cancer, bladder cancer, gastric cancer, ovarian cancer, breast cancer, liver cancer, colon cancer, hematological cancer, or melanoma.

In some embodiments, the cancer is breast cancer, for example, ductal carcinoma in situ, lobular carcinoma in situ, invasive or infiltrating ductal carcinoma, invasive or infiltrating lobular carcinoma, inflammatory breast cancer, triple negative breast cancer, Paget's disease of the nipple, phyllodes tumor, angiosarcoma, or invasive breast cancer.

In some embodiments, the cancer is colon cancer, for example, adenocarcinoma, gastrointestinal carcinoid, gastrointestinal stromal tumor, primary colorectal lymphoma, leiomyosarcoma, squamous cell carcinoma, mucinous adenocarcinoma, or signet ring cell adenocarcinoma.

In some embodiments, the cancer is hematological cancer, for example, leukemia, lymphoma, myeloma, non-Hodgkin's lymphoma, Hodgkin's lymphoma, or B cell lymphoma such as B cell malignancies.

In some embodiments, the cancer is lung cancer, for example, non-small cell lung cancer or small cell lung cancer.

In some embodiments, the cancer is recurrent, refractory, or metastatic cancer.

In some embodiments, the pharmaceutical composition of the multi-kinase inhibitor compound shown in formula (I) or the pharmaceutically acceptable salt, the stereoisomer or the crystal form thereof and the PD-1/PD-L1 inhibitor may be used in combination in a single formulation or administered to a patient as independent agents simultaneously, sequentially, or intermittently, and the route of administration may be oral, parenteral, rectal, or transpulmonary administration.

In some embodiments of the pharmaceutical composition, kit, use, and method of the present invention, the programmed cell death protein 1/programmed cell death protein 1 ligand 1 (PD-1/PD-L1) antibody is a monoclonal antibody.

In some embodiments of the pharmaceutical composition, kit, use, and method of the present invention, the PD-1 antibody is selected from any one or more of monoclonal antibody products having anti-PD-1 effects.

In some embodiments, the PD-1 antibody is a murine antibody, for example, InVivoMAb anti-mouse PD-1 with a clone number of RMP1-14.

In some embodiments of the pharmaceutical composition, kit, use, and method of the present invention, the PD-L1 antibody is selected from any one or more of monoclonal antibody products having anti-PD-L1 effects.

In some embodiments, the PD-L1 antibody may also be a murine antibody, for example, InVivoMAb anti-mouse PD-L1 (B7-H1) with a clone number of 10F.9G2 (IgG2b).

Both the single formulation and the independent agents can be prepared into a suitable dosage form by combining with suitable carriers or excipients based on the properties of the medicament. For example, when used for oral administration, the pharmaceutical composition can be prepared into a conventional solid formulation, such as tablets, capsules, pills, and granules; or can be prepared into an oral liquid formulation, such as oral solutions, oral suspensions, and syrups. In the preparation of an oral formulation, an appropriate filler, binder, disintegrant, lubricant, and the like may be added. For parenteral administration, the pharmaceutical composition can be formulated into an injection, including a solution injection, a sterile powder for injection, and a concentrated solution for injection. The injection can be produced by a conventional method existing in the pharmaceutical field, and during the preparation process, no additive may be added, or an appropriate additive may be added according to the properties of the medicament. For rectal administration, the pharmaceutical composition can be formulated into a suppository and the like. For transpulmonary administration, the pharmaceutical composition can be formulated into an inhalant, spray, or the like.

The present invention also provides a method for treating a cancer using the pharmaceutical composition or the kit of the present invention, which comprises administering to a patient in need a therapeutically effective amount of the active ingredients in the pharmaceutical composition or the kit of the present invention.

The "therapeutically effective amount" described herein refers to an amount of the pharmaceutical composition or the kit that, when administered to a patient, is at least capable of alleviating the symptoms of the patient's condition to treat cancer. An actual amount comprising the "therapeutically effective amount" will vary depending on a variety of circumstances, including, but not limited to, the particular condition being treated, the severity of the condition, the physique and health status of the patient, and the route of administration. The appropriate amount can be readily determined by skilled medical practitioners using methods known in the medical field.

The term "pharmaceutical composition" refers to a mixture or combination of more than one active ingredient and includes both fixed and non-fixed combinations of active ingredients. The term "fixed combination" means that the active ingredients, for example, a compound of formula I and one or more components, are all administered to a patient simultaneously in the form of a single entity or dosage. The term "non-fixed combination" means that the active ingredients, for example, a compound of formula I and one or more components, are administered to a patient as separate entities either simultaneously, separately, or sequentially (without specific time limitation). The latter also applies to a highly active antiretroviral therapy, for example, the administration of three or more active ingredients.

The "subject" or "individual" in the prevention or treatment of cancer refers to any animal classified as a mammal, including humans, domestic animals, and farm animals, as well as zoo, sport, or pet animals, such as dogs, horses, cats, and cattle. Preferably, the mammal is a human. The subject or individual may be a patient.

The term "treat, treating, or treatment" refers to administering to a patient an effective amount of a medicament that slows or cures an undesired physiological change or condition in the patient, such as a hyperproliferative condition, such as the growth, formation, or spread of a cancer, to achieve a favorable or desired clinical result, including, but not limited to: alleviation of symptoms, diminishment of the disease, stabilization (i.e., not worsening) of the disease state, delay or slowing of the disease progression, amelioration or palliation of the disease state, and regression (whether partial or complete) of the disease, whether detectable or undetectable.

The term "treat, treating, or treatment" may include neoadjuvant treatment and adjuvant treatment.

The "neoadjuvant treatment" refers to systemic treatment for a patient with an original tumor prior to planned surgery or local treatment with surgery plus radiotherapy. Treatment means of the neoadjuvant treatment may be chemotherapy, endocrine, targeting, immunization, and radiotherapy depending on the tumor types. The purpose of neoadjuvant treatment is to provide immediate systemic treatment, thereby potentially eradicating micrometastases that otherwise proliferate when following the standard sequence of surgery followed by systemic treatment. The neoadjuvant treatment may also help reduce tumor size, allowing complete resection of an initially unresectable tumor or preservation of portions of the organ and its functions. In addition, the neoadjuvant treatment allows for *in vivo* assessment of the efficacy of a medicament, which may guide the choice of subsequent treatments.

The "adjuvant treatment" refers to a treatment given after definitive surgery (where evidences of residual diseases cannot be detected) to destroy any residual cancer cells in a body, for use in the reduction of the likelihood of tumor relapse or dissemination to other sites. Treatment means of the adjuvant treatment are about the same as those of the neoadjuvant treatment. The objective of the adjuvant treatment is to prevent cancer relapse and thus reduce the chance of cancer-related death. The adjuvant treatment specifically excludes the neoadjuvant treatment herein.

The term "effective to combination therapy" means that a synergistic effect is exhibited when the compounds are administered separately or together (simultaneously, independently, or separately within a time interval) to treat a subject in need thereof, for example, a warm-blooded animal, especially a human.

The term "synergistic effect" refers to an effect produced by two active ingredients (for example, a compound of formula I and the anti-PD-L1 antibody), whose positive effects include: an effect greater than an additive effect, additional beneficial effects (e.g., additional therapeutic effects not previously observed in either ingredient), reduced side effects, a combined therapeutic effect produced by a non-effective dosage of one or both of the first and second active ingredients, and a more synergistic effect of the two active ingredients. The positive effects of the synergistic effect are not limited thereto, and other positive effects may also be included.

### DETAILED DESCRIPTION OF THE INVENTION

The "halogen" described herein refers to fluorine, chlorine, bromine, iodine, and the like, and preferably fluorine and chlorine.

The "halogenated" described herein means that any hydrogen atom in a substituent can be substituted with one or more identical or different halogen atoms. "Halogen" is defined as above.

The "C₁₋₆ alkyl" described herein refers to linear or branched alkyl derived by removing one hydrogen atom from a hydrocarbon moiety containing 1 to 6 carbon atoms, such as methyl, ethyl, *n*-propyl, isopropyl, *n*-butyl, isobutyl, *sec*-butyl, *tert*-butyl, *n*-pentyl, isopentyl, 2-methylbutyl, neopentyl, 1-ethylpropyl, *n*-hexyl, isohexyl, 4-methylpentyl, 3-methylpentyl, 2-methylpentyl, 1-methylpentyl, 3,3-dimethylbutyl, 2,2-dimethylbutyl, 1,1-dimethylbutyl, 1,2-dimethylbutyl, 1,3-dimethylbutyl, 2,3-dimethylbutyl, 2-ethylbutyl, and 1-methyl-2-methylpropyl. The "C₁₋₄ alkyl" refers to the aforementioned examples containing 1 to 4 carbon atoms.

The "C₁₋₆ alkoxy" described herein refers to a group in which the "C₁₋₆ alkyl" defined above is linked to a parent via an oxygen atom, i.e., a "C₁₋₆ alkyl-O-" group, such as methoxy, ethoxy, propoxy, isopropoxy, *n*-butoxy, *tert*-butoxy, *n*-pentyloxy, neopentyloxy, and *n*-hexyloxy. The "C₁₋₄ alkoxy" refers to the aforementioned examples containing 1 to 4 carbon atoms, i.e., "C₁₋₄ alkyl-O-" group.

The "C₁₋₆ alkylsulfonyl" and "C₁₋₆ alkylcarbonyl" described herein refer to C₁₋₆ alkyl-S(O)₂- and C₁₋₆ alkyl-C(O)-, respectively, wherein the "C₁₋₆ alkyl" is defined as above, and is preferably "C₁₋₄ alkyl".

The "C₃₋₆ cycloalkyloxy", "C₃₋₆ cycloalkylamino", "C₃₋₆ cycloalkylsulfonyl", and "C₃₋₆ cycloalkylcarbonyl" described herein refer to groups formed by linking C₃₋₆ cycloalkyl to O, NH, S(O)₂, and C(O), respectively.

The "cycloalkyl", "aryl", "heterocyclyl", and "heteroaryl" described herein include monocyclic and fused ring systems (bicyclic or polycyclic systems). The monocyclic refers to that the group is present in the form of only one ring, and the fused ring refers to that a polycyclic structure is formed by two or more rings linked by an ortho-fused, spiro-, or bridged linkage. The ortho-fused ring refers to a fused ring structure formed by two or more ring structures sharing two adjacent ring atoms (i.e., sharing a bond) with each other. The bridged ring refers to a fused ring structure formed by two or more ring structures sharing two non-adjacent ring atoms with each other. The spiro-ring refers to a fused ring structure formed by two or more ring structures sharing a ring atom with each other. Unless otherwise specified, the cycloalkyl, aryl, heterocyclyl, and heteroaryl defined by the number of atoms in the present invention include monocyclic and fused ring structures which can be formed.

The "cycloalkyl" described herein refers to a monocyclic cycloalkyl or bicyclic cycloalkyl system or a polycyclic cycloalkyl system. These groups may be saturated or unsaturated, but not aromatic. The monocyclic cycloalkyl may be C₃₋₈ cycloalkyl, C₃₋₆ cycloalkyl, C₅₋₈ cycloalkyl, etc., and examples thereof include, but are not limited to: cyclopropyl, cyclobutyl, cyclopentyl, cyclohexyl, cycloheptyl, cyclooctyl, cyclobutenyl, cyclopentenyl, cyclohexenyl, 1,4-cyclohexadienyl, cycloheptenyl, 1,4-cycloheptadienyl, cyclooctenyl, 1,5-cyclooctadienyl, etc. Ortho-fused cycloalkyl may be C₆₋₁₂ membered ortho-fused cycloalkyl or C₇₋₁₀ membered ortho-fused cycloalkyl, and representative examples thereof include, but are not limited to, bicyclo[3.1.1]heptane, bicyclo[2.2.1]heptane, bicyclo[2.2.2]octane, bicyclo[3.2.2]nonane, bicyclo[3.3.1]nonane, and bicyclo[4.2.1]nonane. Spiro-cycloalkyl may be C₆₋₁₂ membered spiro-cyclyl, C₇₋₁₁ membered spiro-cyclyl, etc., and examples thereof include, but are not limited to: 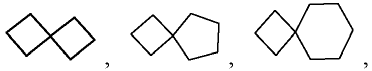 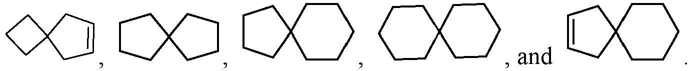 The bridged cycloalkyl may be C₆₋₁₂ membered bridged cyclyl, C₇₋₁₁ membered bridged cyclyl, etc., and examples thereof include, but are not limited to: 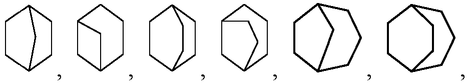 and

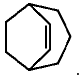

Unless otherwise specified, the "C₃₋₁₄ membered cycloalkyl", "C₃₋₁₀ membered cycloalkyl", and "C₃₋₆ membered cycloalkyl" described herein include monocyclic and fused ring structures which can be formed.

The "heterocyclyl" described herein refers to a non-aromatic cyclic group in which at least one ring carbon atom is substituted with a heteroatom selected from O, S, and N, and preferably 1 to 3 heteroatoms. Moreover, the case where carbon atoms, nitrogen atoms, and sulfur atoms may be oxidized is included.

"Heterocyclyl" refers to a monocyclic heterocyclyl or bicyclic heterocyclyl system or a polycyclic heterocyclyl system, including saturated and partially saturated heterocyclyl, but not including aromatic rings. Monocyclic heterocyclyl may be 3-8 membered heterocyclyl, 3-8 membered saturated heterocyclyl, 3-6 membered heterocyclyl, 4-7 membered heterocyclyl, 5-7 membered heterocyclyl, 5-6 membered heterocyclyl, 5-6 membered oxygen-containing heterocyclyl, 5-6 membered nitrogen-containing heterocyclyl, 5-6 membered saturated heterocyclyl, etc. Examples of "monocyclic heterocyclyl" include, but are not limited to, azacyclopropyl, ozacyclopropyl, thiocyclopropyl, azacyclobutyl, oxacyclobutyl, thiocyclobutyl, tetrahydrofuranyl, tetrahydropyrrolyl, tetrahydrothienyl, imidazolidinyl, pyrazolidinyl, 1,2-oxazolidinyl, 1,3-oxazolidinyl, 1,2-thiazolidinyl, 1,3-thiazolidinyl, tetrahydro-2*H*-pyranyl, tetrahydro-2*H*-thiopyranyl, piperidinyl, piperazinyl, morpholinyl, 1,4-dioxanyl, and 1,4-oxathianyl. Examples of "partially saturated heterocyclyl" include, but are not limited to, 4,5-dihydroisoxazolyl, 4,5-dihydrooxazolyl, 2,5-dihydrooxazolyl, 2,3-dihydrooxazolyl, 3,4-dihydro-2*H*-pyrrolyl, 2,3-dihydro-1*H*-pyrrolyl, 2,5-dihydro-1*H*-imidazolyl, 4,5-dihydro-1*H*-imidazolyl, 4,5-dihydro-1*H*-pyrazolyl, 4,5-dihydro-3*H*-pyrazolyl, 4,5-dihydrothiazolyl, 2,5-dihydrothiazolyl, 2*H*-pyranyl, 4*H*-pyranyl, 2*H*-thiopyranyl, 4*H*-thiopyranyl, 2,3,4,5-tetrahydropyridyl, 1,2-isoxazolyl, 1,4-isoxazolyl, 6*H*-1,3-oxazinyl, etc. Fused heterocyclyl includes ortho-fused heterocyclyl, spiro-heterocyclyl, and bridged heterocyclyl, which may be saturated, partially saturated, or unsaturated, but non-aromatic. Fused heterocyclyl is 5 or 6 membered monocyclic heterocyclyl ring which is fused to benzene ring, 5 or 6 membered monocyclic cycloalkyl, 5 or 6 membered monocyclic cycloalkenyl, 5 or 6 membered monocyclic heterocyclyl, or 5 or 6 membered monocyclic heteroaryl. The ortho-fused heterocyclyl may be 6-12 membered ortho-fused cyclyl, 7-11 membered ortho-fused cyclyl, 6-10 membered ortho-fused cyclyl, 6-12 membered saturated ortho-fused cyclyl, or 7-11 membered saturated ortho-fused cyclyl, and examples thereof include, but are not limited to: 3-azabicyclo[3.1.0]hexyl, 3,6-diazabicyclo[3.2.0]heptyl, 3,8-diazabicyclo[4.2.0]octyl, 3,7-diazabicyclo[4.2.0]octyl, octahydropyrrolo[3,4-*c*]pyrrolyl, octahydropyrrolo[3,4-*b*]pyrrolyl, octahydropyrrolo[3,4-*b*][1,4]oxazinyl, octahydro-1*H*-pyrrolo[3,4-c]pyridyl, 2,3-dihydrobenzofuran-2-yl, 2,3-dihydrobenzofuran-3-yl, indolin-1-yl, indolin-2-yl, indolin-3-yl, 2,3-dihydrobenzothiophen-2-yl, octahydro-1*H*-indolyl, and octahydrobenzofuranyl.

The spiro-heterocyclyl may be 6-12 membered spiro-heterocyclyl, 7-11 membered spiro-heterocyclyl, 7-11 membered saturated spiro-heterocyclyl, or 6-12 membered saturated spiro-cyclyl, and examples thereof include, but are not limited to:

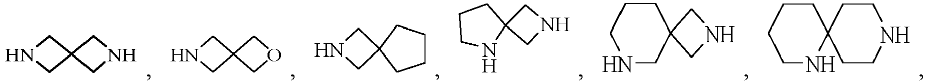

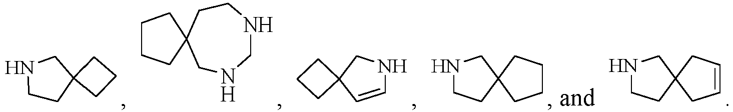

The bridged heterocyclyl may be 6-12 membered bridged heterocyclyl, 7-11 membered bridged heterocyclyl, 6-12 membered saturated bridged cyclyl, or 7-11 membered saturated bridged heterocyclyl, and examples thereof include, but are not limited to: 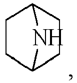 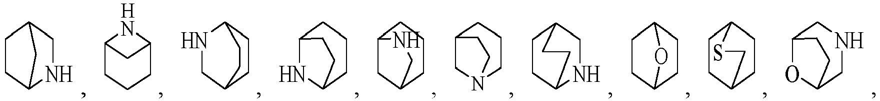 and

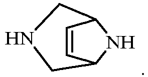

Unless otherwise specified, the 5-14 membered heterocyclyl, 5-11 membered heterocyclyl, 5-10 membered heterocyclyl, 6-10 membered heterocyclyl, 7-11 membered heterocyclyl, and 7-11 membered saturated heterocyclyl described herein include monocyclic and fused ring structures which can be formed.

The aryl described herein refers to an aromatic cyclic group, including monocyclic, bicyclic, or polycyclic systems, which may be 6-14 membered aryl, including "6-8 membered monocyclic aryl", for example, phenyl, cyclooctenyl, etc.; including "8-14 membered fused ring aryl", for example, pentalenyl, naphthyl, phenanthryl, etc.

The term "heteroaryl" used herein refers to an aromatic cyclic group in which at least one ring carbon atom is substituted with a heteroatom selected from O, S, and N, and preferably 1 to 3 heteroatoms. Moreover, the case where carbon atoms or sulfur atoms are oxidized is included. For example, carbon atoms are substituted with C(O), S(O), or S(O)₂. Heteroaryl includes monocyclic heteroaryl and fused heteroaryl, which may be 5-14 membered heteroaryl, 5-10 membered heteroaryl, 5-7 membered heteroaryl, 5-6 membered heteroaryl, or 8-10 membered heteroaryl, and representative examples of the monocyclic heteroaryl include, but are not limited to, furyl, imidazolyl, isoxazolyl, thiazolyl, isothiazolyl, oxadiazolyl, oxazolyl, isoxazolyl, pyridyl, pyridazinyl, pyrimidinyl, pyrazinyl, pyrazolyl, pyrrolyl, tetrazolyl, thiadiazolyl, thiazolyl, thienyl, triazolyl, and triazinyl. Fused heteroaryl refers to a bicyclic or polycyclic system fused to phenyl, cycloalkyl, cycloalkenyl, heterocyclyl, or heteroaryl. Fused heteroaryl may be 8-12 membered heteroaryl, 8-10 membered heteroaryl, or 9-10 membered heteroaryl, and representative examples thereof include, but are not limited to, benzimidazolyl, benzofuranyl, benzothienyl, benzoxadiazolyl, benzothiazolyl, cinnolinyl, 5,6-dihydroquinolin-2-yl, 5,6-dihydroisoquinolin-1-yl, indazolyl, indolyl, isoquinolinyl, naphthyridinyl, purinyl, quinolinyl, 5,6,7,8-tetrahydroquinolin-2-yl, 5,6,7,8-tetrahydroquinolinyl, 5,6,7,8-tetrahydroquinolin-4-yl, 5,6,7,8-tetrahydroisoquinolin-1-yl, 4,5,6,7-tetrahydro[c][1,2,5]oxadiazolyl, and 6,7-dihydro[c][1,2,5]oxadiazol-4(5*H*)keto. In certain embodiments, fused heteroaryl is 5 or 6 membered monocyclic heteroaryl ring which is fused to benzene ring, 5 or 6 membered monocyclic cycloalkyl, 5 or 6 membered monocyclic cycloalkenyl, 5 or 6 membered monocyclic heterocyclyl, or 5 or 6 membered monocyclic heteroaryl.

Unless otherwise specified, the 5-14 membered heteroaryl, 5-10 membered heteroaryl, 6-10 membered heteroaryl, 5-6 membered heteroaryl, and 8-10 membered heteroaryl described herein include monocyclic and fused ring structures which can be formed.

The term "PD-1" refers to programmed cell death protein 1.

The term "PD-L1" refers to programmed cell death protein 1 ligand 1.

The term "antibody" refers to an antigen-binding protein having at least one antigen-binding domain. An antibody molecule binds to a specific antigenic determinant or an epitope on an antigen with the aid of a specific binding site. The antibody molecule consists of four polypeptides (two identical heavy chains and two identical light chains) linked to form a "Y" shaped molecule. Each heavy chain consists of a heavy chain variable region (V_{H}) and a heavy chain constant region. The heavy chain constant region consists of three or four constant domains (CH₁, CH₂, CH₃, and CH₄, depending on the antibody types or isotypes). Each light chain consists of a light chain variable region (V_{L}) and a light chain constant region C_{L} having one domain. The V_{L} and V_{H} regions are located at the terminals of the "Y" shaped antibody molecule.

The term "variable region" or "variable domain" refers to a domain of an antibody heavy or light chain that is involved in the binding of an antigen-binding molecule to an antigen. The heavy and light chain variable domains (V_{H} and V_{L}, respectively) of natural antibodies generally have similar structures, with each domain containing four conserved framework regions (FRs) and three hypervariable regions (HVRs). See, e.g., Kindt et al., *Kuby Immunology,* 6^{th} edition, W. H. Freeman and Co., Page 91 (2007). A single V_{H} or V_{L} domain may be sufficient to provide antigen-binding specificity.

The term "hypervariable region" or "HVR" refers to a region in an antibody variable domain that is hypervariable in sequence ("complementarity determining region" or "CDR") and/or forms a structurally defined loop ("hypervariable loop") and/or comprises antigen-contacting residues ("antigen-contacting sites").

The "antibody" of the present invention refers to any form of an antibody that exhibits the desired biological or binding activity. Thus, it is used in the broadest sense and specifically covers, but is not limited to, a monoclonal antibody (including a full-length monoclonal antibody), a polyclonal antibody, a multispecific antibody (for example, a bispecific antibody), a humanized and primatized antibody, a fully human antibody, a chimeric antibody, a murine antibody, and a camelized single-domain antibody. "Parental antibody" is an antibody obtained by exposure of an immune system to an antigen prior to modification of the antibody for intended use, such as humanization of a parental antibody generated in a mouse for use as a human therapeutic.

The PD-1 antibody or PD-L1 antibody of the present invention may be a murine antibody, a chimeric antibody, a humanized antibody, or a fully human antibody.

The term "murine antibody" refers to an antibody produced by mice against a specific antigen, generally referring to an antibody produced by a mouse B lymphocyte. In most cases, the murine antibody is a monoclonal antibody produced by a hybridoma cell.

The term "chimeric antibody" refers to an antibody in which a portion of the heavy chain and/or light chain is identical with or homologous to corresponding sequences in an antibody derived from a particular species (for example, human) or belonging to a particular antibody class or subclass, while the remainder of the chain(s) is identical with or homologous to corresponding sequences in an antibody derived from another species (for example, mouse) or belonging to another antibody class or subclass, as well as fragments of such antibodies, so long as they exhibit the desired biological activity.

The term "fully human antibody" refers to an antibody that comprises human immunoglobulin protein sequences only. A fully human antibody may contain murine carbohydrate chains if produced in a mouse, in a mouse cell, or in a hybridoma derived from a mouse cell. Similarly, "mouse antibody" or "rat antibody" refers to an antibody that comprises only mouse or rat immunoglobulin sequences, respectively.

The term "humanized antibody" means that the antigen-binding site in the antibody is derived from a non-human species and the variable region framework is derived from human immunoglobulin sequences. A humanized antibody may comprise substitutions in the framework region such that the framework may not be an exact copy of the expressed human immunoglobulin or germline gene sequence.

The term "monoclonal antibody" (mAb) refers to an antibody molecule composed of a single molecule. A monoclonal antibody composition exhibits a single binding specificity and affinity for a particular epitope or, in the case of a bispecific monoclonal antibody, a dual binding specificity for two different epitopes. MAb is an example of an isolated antibody. MAbs can be produced by hybridoma techniques, recombinant techniques, transgenic techniques, or other techniques known to those of skill in the art.

The term "PD-1 antibody" refers to any antibody that specifically binds to programmed cell death protein 1 (PD-1) or a soluble fragment thereof and blocks the binding of a ligand to PD-1, thereby causing competitive inhibition of PD-1 and inhibition of PD-1-mediated T cell activation.

An exemplary PD-1 antibody can be InVivoMAb anti-mouse PD-1 with a clone number of RMP1-14.

The term "PD-L1 antibody" refers to an antibody that specifically binds to programmed cell death protein 1 ligand 1 (PD-L1) or a soluble fragment thereof and blocks the binding of a ligand to PD-1, thereby causing competitive inhibition of PD-1 and inhibition of PD-1-mediated T cell activity.

An exemplary PD-L1 antibody can be InVivoMAb anti-mouse PD-L1 (B7-H1) with a clone number of 10F.9G2 (IgG2b).

### BRIEF DESCRIPTION OF THE DRAWINGS

In order to more clearly illustrate the embodiments of the present invention and the technical schemes of the prior art, the drawings used in the embodiments and the prior art are briefly introduced below. It is obvious that the drawings in the following description are only some embodiments of the present invention, and that other drawings can be obtained by those of ordinary skill in the art without creative efforts.
FIG. 1 is an individual graph of the effect of the combination with PD-1 antibody on tumor growth in CT26.wt tumor-bearing mice.
FIG. 2 is a western blot exposure map of the regulation of IDO protein levels in Hela cells.

### DETAILED DESCRIPTION

The present invention can be better understood according to the following examples. However, it is easily understood by those skilled in the art that the contents described in the examples are only used to illustrate the present invention, and should not and will not limit the present invention. The references to method of treatment in this description are to be interpreted as references to the products of the present invention for use in those methods of treatment.

### List of abbreviations

Qd: once daily; Q3d: once every three days; MC: methylcellulose; DMSO: dimethyl sulfoxide; IP.: intraperitoneal administration; PO: oral administration; QD: once-daily administration; BIW: twice-weekly administration.

### Example 1: Synthesis of intermediate 1-(4-methoxybenzyl)-5-methyl-4-nitro-1H-pyrazol-3-amine

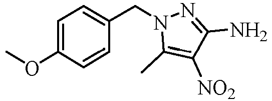

### Step 1: Synthesis ofN-(5-methyl-1H-pyrazol-3-yl)acetamide

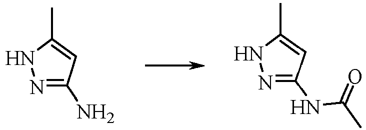

5-Methyl-1*H*-pyrazol-3-amine (300 g, 3.09 mol, 1.0 eq) was weighted into a 5 L round-bottom flask and dissolved by adding water (2800 mL) with mechanical stirring at room temperature. Sodium bicarbonate (780 g, 9.28 mol, 3 eq) was added in batches, followed by stirring for another 30 min after the addition. Then, acetic anhydride (592 mL, 6.2 mol, 2 eq) was added dropwise and slowly to the reaction system over about 1 h at a controlled rate of dropwise addition. At this point, a large amount of foamy white solid was produced. The reaction system was heated to 100 °C and then stirred for 2 h. The solid was gradually dissolved, and the system became clarified. Then heating was stopped, and the system was cooled to room temperature. After stirring overnight, a large amount of white crystalline solid was precipitated. Another batch of 5-methyl-1*H*-pyrazol-3-amine (300 g) was fed in parallel. After the reactions were completed, the two batches were combined and filtered, and the solid was washed with water (500 mL × 2) and dried to give a white solid (554 g, yield 62%).

¹H NMR (400 MHz, DMSO-*d₆*) δ (ppm): 10.32 (s, 1H), 6.21 (s, 1H), 2.18 (s, 3H), 1.97 (s, 3H).

### Step 2: Synthesis of N-(5-methyl-4-nitro-1H-pyrazol-3-yl)acetamide

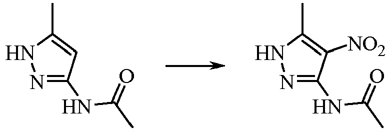

Concentrated sulfuric acid (2 L, about 36.8 mol, 9.2 eq) was charged into a 5 L round-bottom flask and cooled in an ice-water bath, and then *N*-(5-methyl-1*H*-pyrazol-3-yl)acetamide (554 g, 3.98 mol, 1 eq) was added in batches over 1 h with mechanical stirring. Stirring was continued until the solid was completely dissolved. Then fuming nitric acid (250 mL, about 5.7 mol, 1.4 eq) was added dropwise to the system over 2 h at a controlled temperature of less than 15 °C. The reaction was completed as detected by LC-MS after the reaction was continued for another 15 min. The reaction system was slowly poured into 5 L of crushed ice water with mechanical stirring, and a large amount of white solid was precipitated immediately. The mixture was allowed to stand overnight and filtered. The resulting solid was washed with water (1000 mL × 2) and subjected to infrared drying to give a white solid (587 g, yield 80%).

¹H NMR (400 MHz, DMSO-*d₆*) δ (ppm): 10.22 (s, 1H), 2.44 (s, 1H), 2.13 (s, 3H).

### Step 3: Synthesis of 5-methyl-4-nitro-1H-pyrazol-3-amine

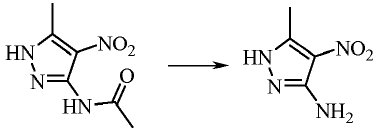

Water (1.1 L) and concentrated hydrochloric acid (1 L, about 12 mol, 4 eq) were charged to a 5 L four-neck round-bottom flask and gradually heated to 80 °C. *N*-(5-Methyl-4-nitro-1*H*-pyrazol-3-yl)acetamide (587 g, 3 mol, 1 eq) was added in batches over about 1 h with mechanical stirring. The mixture was continued to reflux at 100 °C for about 1 h until the system was clarified. The mixture was filtered after cooling to remove the insoluble matter. The filtrate was concentrated under reduced pressure to give a crude product, which was then slurried with methyl *tert*-butyl ether and filtered. The filter cake was dried to give an orange solid (610 g of crude product).

### Step 4: Synthesis of 1-(4-methoxybenzyl)-5-methyl-4-nitro-1H-pyrazol-3-amine

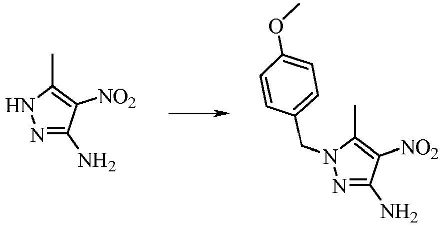

5-Methyl-4-nitro-1*H*-pyrazol-3-amine hydrochloride (200 g, 1.12 mol, 1 eq) was charged into a 3 L round-bottom flask and dissolved by adding DMF (1.8 L) with mechanical stirring at room temperature. Potassium carbonate (335 g, 2.42 mol, 2.1 eq) was added slowly in batches over about 40 min, and then 4-methoxybenzyl chloride (177 g, 1.13 mol, 1 eq) was added dropwise over 30 min. The reaction system was stirred at room temperature overnight. A small amount of starting material remained as detected by TLC and LC-MS. Another two batches of 5-methyl-4-nitro-1*H*-pyrazol-3-amine hydrochloride (200 g, 1.12 mol) were fed in parallel. After the reactions were completed, the mixtures were filtered. The filtrates were combined, concentrated under reduced pressure until half of the solvent remained, and then poured into ice water (about 2.5 L) with stirring. A brownish yellow solid was precipitated, and the mixture was allowed to stand overnight. The filter cake was washed with DCM (1000 mL × 3) and concentrated under reduced pressure, and the residue was poured into ice water (about 1000 mL). A brownish yellow solid was precipitated, and the mixture was allowed to stand overnight. The solids precipitated above were combined, washed with water (500 mL × 2), dried under vacuum, and slurried with ethyl acetate. The mixture was filtered, and the filter cake was dried to give a yellow solid (268 g, yield 30%).

¹H NMR (400 MHz, DMSO-*d₆*) δ (ppm): 7.18 (d, *J* = 8.6 Hz, 2H), 6.90 (d, *J*= 8.6 Hz, 2H), 6.18 (s, 2H), 5.09 (s, 2H), 3.73 (s, 3H), 2.56 (s, 3H).

### Example 2: Synthesis of intermediate (6-bromo-4-iodo-pyridin-3-yl)(2-chlorophenyl)methanone

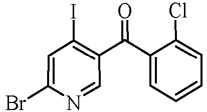

### Step 1: Synthesis of (6-bromopyridin-3-yl)(2-chlorophenyl)methanol

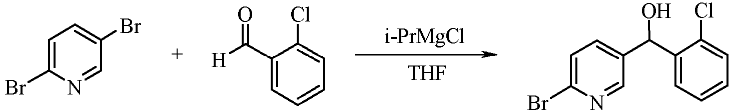

Anhydrous tetrahydrofuran (500 mL) and 2,5-dibromopyridine (100.0 g, 0.42 mol, 1.0 eq) were added into a 2 L four-neck flask, and the mixture was cooled to 2 °C with stirring in an ice-water bath. Isopropyl magnesium chloride (210.5 mL, 2.0 M, 0.42 mol, 1.0 eq) was then added dropwise over about 0.5 h at a controlled temperature of no more than 10 °C. The reaction system was stirred at room temperature (20 °C) for 1 h and then cooled to 10 °C with an ice-water bath. A solution of 2-chlorobenzaldehyde (62.3 g, 0.443 mol, 1.05 eq) in tetrahydrofuran (200 mL) was added dropwise over about 0.5 h. The mixture was stirred at 10 °C for 2 h, and the reaction was completed as indicated by TLC. A saturated aqueous ammonium chloride solution (300 mL) was added into the reaction system. After stirring for 10 min, the organic phase was separated and concentrated to give a yellow oil. The aqueous phase was extracted with ethyl acetate (1.0 L×2). The organic phase was combined with the yellow oil obtained above, washed with water (500 mL) followed by saturated brine (500 mL), dried over anhydrous Na₂SO₄, and concentrated to give a brown oil (140 g of crude product).

### Step 2: Synthesis of (6-bromopyridin-3-yl)(2-chlorophenyl)methanone

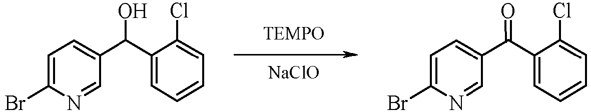

(6-Bromopyridin-3-yl)(2-chlorophenyl)methanol (140 g of crude product) was dissolved in DCM (1.3 L), and TEMPO (1.51 g, 9.4 mmol) and NaBr (1.92 g, 18.8 mmol) were added. The reaction system was cooled to 3 °C in an ice-water bath, and an aqueous NaClO solution (1.34 mol/L, 600 L, 0.71 mol) neutralized with NaHCO₃ (45.0 g) was added dropwise at a controlled temperature of no more than 20 °C. After dropwise addition, the mixture was stirred for 10 min, and the reaction was completed as indicated by TLC. The aqueous phase was separated and extracted with DCM (1.0 L). The organic phases was combined, washed with water (1.0 L) followed by saturated brine (1.0 L), dried over anhydrous Na₂SO₄, and concentrated to give a yellow oil. The crude product was slurried with methyl tert-butyl ether (150 mL)/petroleum ether (500 mL) to give a yellow solid (50.3 g, yield: 39.7% for two steps).

### Step 3: Synthesis of (6-bromo-4-iodo-pyridin-3-yl)(2-chlorophenyl)methanone

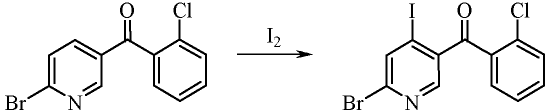

In nitrogen atmosphere, a solution of tetramethylpiperidine lithium/magnesium chloride (281 mL, 1.5 mol/L, 0.43 mol, 2.5 eq) was added into a 2 L four-neck flask and cooled to -65 °C in a dry ice/ethanol bath. A solution of (6-bromopyridin-3-yl)(2-chlorophenyl)methanone (50.0 g, 0.17 mol, 1.0 eq) in tetrahydrofuran (50 mL) was added dropwise over about 0.5 h. Then, the reaction mixture was heated to -45 °C, stirred for 1 h, and then cooled to -65 °C again. A solution of I₂ (129.3 g, 0.51 mol, 3.0 eq) in tetrahydrofuran (400 mL) was added dropwise over about 1 h. The mixture was stirred for 20 min, and the reaction was completed as indicated by TLC. To the reaction system were added a saturated aqueous ammonium chloride solution (500 mL) and a saturated aqueous NaHSO₃ solution (500 mL). The mixture was stirred for 15 min and filtered, and the insoluble matter was washed with ethyl acetate (500 mL × 2). The filtrates were combined, and the aqueous phase was separated and extracted with ethyl acetate (1.0 L × 2). All the organic phases were combined, washed with water (800 mL) followed by saturated brine (800 mL), dried over anhydrous Na₂SO₄, and concentrated to give a yellow solid. The solid was slurried with methyl tert-butyl ether (500 mL)/petroleum ether (500 mL) and dried to give a yellow solid (30 g, yield: 41.8%).

1H NMR (400 MHz, CDCl₃) δ (ppm): 7.38-7.50 (m, 2H), 7.51-7.65 (m, 2H), 8.17 (s, 1H), 8.24 (s, 1H).

### Example 3: Synthesis of intermediate (6-bromo-4-((1-(4-methoxybenzyl)-5-methyl-4-nitro-1H-pyrazol-3-yl)amino)pyridin-3-yl)(2-chlorophenyl)methanone

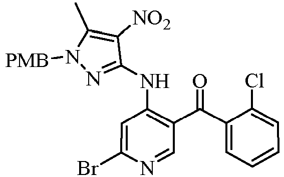

### Step 1: Synthesis of (6-bromo-4-((1-(4-methoxybenzyl)-5-methyl-4-nitro-1H-pyrazol-3-yl)amino)pyridin-3-yl)(2-chlorophenyl)methanone

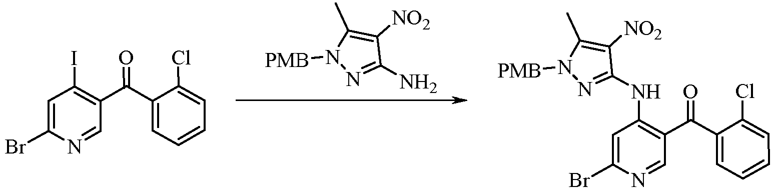

1-(4-Methoxybenzyl)-5-methyl-4-nitro-1*H*-pyrazol-3-amine (14.92 g, 56.9 mmol) was added into anhydrous tetrahydrofuran (100 mL) and dissolved with stirring in nitrogen atmosphere. NaH (40% by mass, 4.82 g, 0.11 mol) was added in batches under an ice bath. The mixture was stirred for 1 h after the addition, and then a solution of (6-bromo-4-iodo-pyridin-3-yl)(2-chlorophenyl)methanone (20 g, 47.4 mmol) in tetrahydrofuran (100 mL) was added dropwise. The mixture was incubated at room temperature for reaction for 16 h, and the reaction was completed as detected by TLC. Methanol (30 mL) was added to quench the reaction, followed by addition of a saturated ammonium chloride solution (50 mL). The resulting mixture was filtered to give a yellow product (25 g, yield 80%).

¹H-NMR (400 MHz, DMSO-*d₆*): 12.36 (s, 1H), 8.77 (s, 1H), 8.11 (s, 1H), 7.66-7.53 (m, 4H), 7.33 (d, J=8.8 Hz, 1H), 7.33 (d, *J*=8.8 Hz, 1H), 6.96 (d, *J*=8.8 Hz, 1H), 5.39 (s, 2H), 3.75 (s, 3H), 2.73(s, 3H).

### Example 4: Synthesis of 4-(5-(2-chlorophenyl)-3-methyl-2,10-dihydropyrazolo[4,3-b]pyrido[4,3-e][1,4]diazepin-8-yl)morpholine (compound 1)

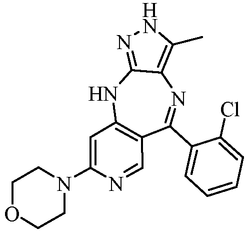

### Step 1: Synthesis of (6-bromo-4-((5-methyl-4-nitro-1H-pyrazol-3-yl)amino)pyridin-3-yl)(2-chlorophenyl)methanone

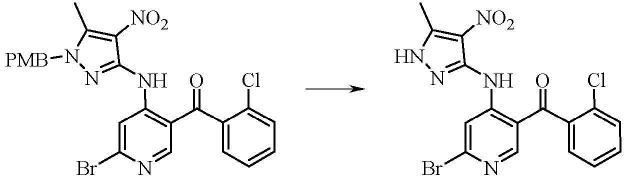

(6-Bromo-4-((1-(4-methoxybenzyl)-5-methyl-4-nitro-1*H*-pyrazol-3-yl)amino)pyridin-3-yl)(2-chlorophenyl)methanone (intermediate 1-4) (0.80 g, 1.4 mmol) was dissolved in DCM (2 mL), and trifluoroacetic acid (10 mL) was added dropwise. The mixture was heated to 70 °C for reaction for 4 h. The reaction was completed as detected by LC-MS. The mixture was then cooled and concentrated under vacuum to give a yellow solid (1.0 g of crude product).

### Step 2: Synthesis of 8-bromo-5-(2-chlorophenyl)-3-methyl-2,10-dihydropyrazolo[4,3-b]pyrido[4,3-e][1,4]diazepine

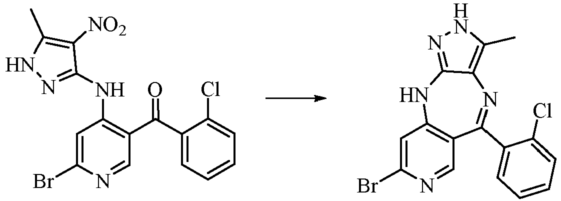

(6-Bromo-4-((5-methyl-4-nitro-1*H*-pyrazol-3-yl)amino)pyridin-3-yl)(2-chlorophenyl)methanone (1.0 g of crude product) was dissolved in 2-methyltetrahydrofuran (15 mL), and Tin(II) chloride dihydrate (3.2 g, 14.2 mmol) was added. The mixture was heated to 100 °C for reaction for 16 h. The reaction was completed as indicated by LC-MS. An aqueous sodium hydroxide solution was added to adjust pH to 10, and the mixture was filtered through celite, extracted with 2-methyltetrahydrofuran, and concentrated. The crude product was purified by silica gel column chromatography (dichloromethane:methanol = 30: 1) to give the product (60 mg, two-step yield 10.7%).

### Step 3: Synthesis of 4-(5-(2-chlorophenyl)-3-methyl-2,10-dihydropyrazolo[4,3-b]pyrido[4,3-e][1,4]diazepin-8-yl)morpholine

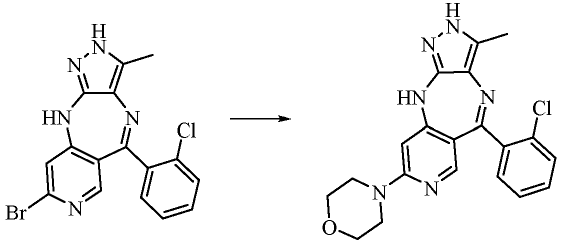

8-Bromo-5-(2-chlorophenyl)-3-methyl-2,10-dihydropyrazolo[4,3-*b*]pyrido[4,3-*e*][1,4]diazepine (60 mg, 0.16 mmol) was dissolved in DMSO (2 mL), and morpholine (14 mg, 0.20 mmol) was added in nitrogen atmosphere. The mixture was heated to 110 °C for reaction for 6 h. The reaction was completed as indicated by LC-MS. The reaction solution was poured into water (10 mL), extracted with dichloromethane (20 mL × 2), and concentrated. The crude product was purified by silica gel column chromatography (dichloromethane:methanol = 20:1) to give the product (16 mg, yield: 25%).

¹H-NMR (400 MHz, DMSO-*d₆*): 11.56 (s, 1H), 8.29 (s, 1H), 7.33-7.47 (m, 4H), 6.88 (s, 1H), 5.96 (s, 1H), 3.62 (m, 4H), 3.33 (m, 4H), 1.97 (s, 3H).

Molecular formula: C₂₀H₁₉ClN₆O molecular weight: 394.86 LC-MS (Pos, *m*/*z*) = 394.96 [M+H⁺].

Example 5: *In vivo* pharmacodynamic study of the compound of the present invention combined with PD-1 antibody on mouse colon cancer cell CT26.wt subcutaneous homograft tumor model

Test sample: compound 1 of the present invention, having a structure shown above.

Animals, cells, reagents & instruments:
CT26.wt cells: mouse colon cancer cells from the Cell Bank of the Chinese Academy of Sciences.
Balb/c mice, 6- to 8-week-old, female, from Beijing Vital River Laboratory Animal Technology Co., Ltd.
PD-1 antibody: InVivoMAb anti-mouse PD-1, clone number: RMP1-14, company: BioXcell, Cat. No. BE0146.

### Test method:

### (1) Construction and grouping of tumor-bearing mice

CT26.wt cells were cultured in monolayer *in vitro* under the condition of an RPMI1640 medium supplemented with 10% heat-inactivated fetal bovine serum and 1% penicillin-streptomycin double antibody, and the culture was carried out at 37 °C and 5% CO₂. Subculture treatment was carried out in a 1:3 ratio. When the cells were in the exponential growth phase, the cells were harvested, counted, and inoculated.

0.1 mL of a cell suspension containing about 1 × 10⁶ CT26.wt cells (cells suspended in serum-free basal RPMI1640 medium) was inoculated subcutaneously into the left back skin of female Balb/c mice. The mice were grouped for administration when the average volume of tumors reached approximately 100 mm³. Grouping method: Animals were weighed before administration and tumor volumes were measured. Grouping was designed in blocks according to the tumor volume, with 8 mice per group.

### (2) Administration regimen

Administration was carried out as in Table 2 below.

**Table 2. Administration mode**

| Group | Dosage (mg/kg) | Route of administration | Frequency of administration | Administration period |
|---|---|---|---|---|
| Vehicle control | 0 | PBS: IP. | PBS: BIW | 1-14 days |
| | | 0.5%MC: PO. | 0.5%MC: QD. | |
| Compound 1 | 15 | PO. | QD. | 1-14 days |
| PD-1 antibody | 10 | IP. | BIW. | 1-14 days |
| Compound 1 | 15 | Compound 1: PO. | Compound 1: QD. | 1-14 days |
| PD-1 antibody | 10 | PD-1 antibody: IP. | PD-1 antibody: BIW. | 1-14 days |

| | | | | |
|---|---|---|---|---|
| IP.: intraperitoneal administration; PO.: oral administration; QD.: once-daily administration; BIW: twice-weekly administration. | | | | |

### (3) Experimental observation index

Animals were monitored daily for health status and mortality, body weight and tumor volume were measured twice a week, and samples were collected after the last administration. The therapeutic effect of tumor volume was evaluated by TGI%, wherein the relative tumor inhibition rate TGI (%): TGI = 1-T/C (%). T/C % is the relative tumor proliferation rate, i.e., the percentage of the relative tumor volume in the treatment and control groups at a given time point. T and C are relative tumor volumes (RTVs) of the treatment and control groups at a given time point, respectively. The calculation formula is as follows: T/C % = T_{RTV} / C_{RTV} × 100% (T_{RTV}: mean RTV for treatment group; C_{RTV}: mean RTV for vehicle control group; RTV = Vₜ / V₀, wherein V₀ is the tumor volume of the animal at the time of grouping, and Vₜ is the tumor volume of the animal after treatment).

### (4) Results

**Table 3. Effect of the combination with PD-1 antibody on tumor growth in CT26.wt tumor-bearing mice (day 13)**

| Group | Number of animals | Tumor volume (mm³) | TGI (%)^{a} |
|---|---|---|---|
| Vehicle control | 8 | 2202 ± 247 | -- |
| Compound 1 | 8 | 1150 ± 142 | 48 |
| PD-1 antibody | 8 | 1771 ± 223 | 19 |
| Compound 1 + PD-1 antibody | 8 | 754 ± 108 | 66 |

| | | | |
|---|---|---|---|
| Notes: ^{a}: TGI: tumor volume inhibition rate. | | | |

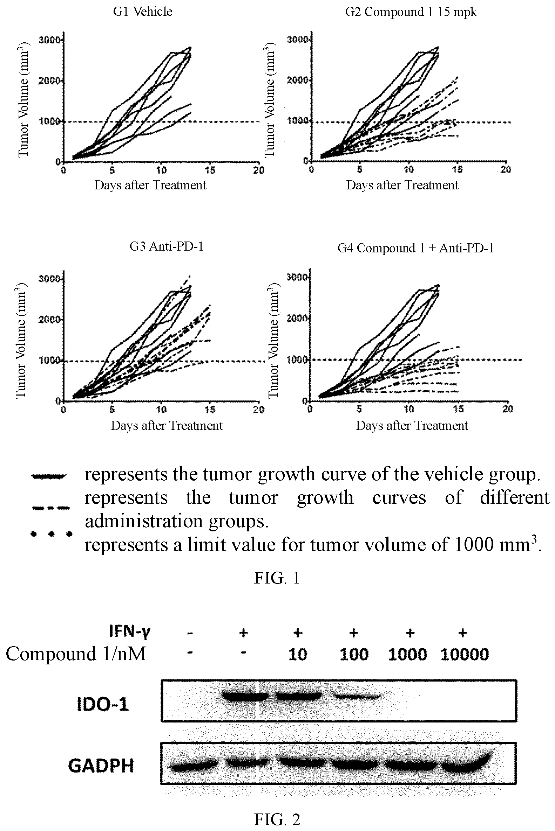

As can be seen from Table 3 and FIG. 1, the combination of the compound of the present invention with PD-1 antibody had a significant inhibitory effect on the CT26.wt cell homograft tumor model, and had an obvious effect of enhancing the efficacy by the combination, indicating that the combination of the compound of the present invention with PD-1 antibody has good clinical application potential for immuno-oncology therapy.

### Example 6: Study on regulation of IDO protein levels in Hela cells by the compound of the present invention

### (1) Cells, reagents & instruments

Cells: Hela cells from Cobioer.

Reagents: DMEM medium, fetal bovine serum, penicillin, streptomycin, interferon-γ (IFN-γ) from SinoBiological, protein loading buffer, RIPA protein lysis buffer, protease inhibitor, BCA protein content assay kit, trihydroxymethyl aminomethane, sodium dodecyl sulfate, ammonium persulfate, 30% Acry-Bis solution, phosphate buffer, Tween 20, bovine serum albumin, GAPDH antibody (rabbit) from CST company, IDO-1 antibody (rabbit) from CST, anti-rabbit secondary antibody from CST, and protein marker.

Instruments: refrigerated centrifuge, orbital vibrator, Nanodrop ultramicro nucleic acid analyzer, membrane transfer apparatus, electrophoresis apparatus, and gel imaging system.

### (2) Study method

Cell culture and plating: Hela cells were cultured in a DMEM medium containing 10% fetal bovine serum and 1% penicillin-streptomycin double antibody and incubated in a water-jacketed incubator at 37 °C and 5% CO₂. Subculture treatment was carried out in a 1:4 ratio. When the cells were in the exponential growth phase, the cells were harvested, counted, plated at a density of 5 × 10⁵ cells/well in a 6-well cell culture plate, and cultured overnight.

Compound dilution: Compound 1 was dissolved in DMSO to prepare a 10 mM stock solution, and then diluted with DMSO in a 10-fold gradient for 3 concentrations (1 mM, 0.1 mM, and 0.01 mM) to prepare a 1000× diluted compound mother liquor.

Incubation with compound: After cell inoculation and culture overnight, 1 µL of corresponding diluted compound mother liquor (1000×) was added into each well to obtain 4 dosing wells (with DMSO content being 1 ‰) with final concentrations of 10000 nM, 1000 nM, 100 nM, and 10 nM, respectively. 1 µL of DMSO was added into the positive and negative control wells, and interferon-γ with a final concentration of 50 ng/mL was simultaneously added into the dosing wells and positive control wells. Then, co-incubation was carried out for 16 h.

Collection of cell total protein: After incubation was completed, the cells in each group were collected into a centrifuge tube, washed twice with pre-cooled PBD, and then placed on ice. 40 µL of pre-cooled RIPA protein lysis buffer containing protease inhibitor was added into each well. The mixture was shaken for lysis for 30 min and centrifuged at 12000 rpm for 10 min, and the supernatant was collected.

Protein content assay and sample configuration: Protein content in each well was measured by using a BCA kit and Nanodrop, and according to the detection result, a sample loading buffer and a lysis buffer were added to adjust to a system of 20 µg of protein/10 µL for sample loading of Western Blot detection.

Western blot detection: A 10% separation gel and a 5% concentration gel were used as electrophoresis conditions, and a constant pressure of 100 V and a duration of 120 min were used as membrane transfer conditions. After membrane transfer, incubation was carried out for 1 h at room temperature with 5% skimmed milk powder and then continued with a primary antibody overnight at 4 °C with uniform shaking. After washing the membrane, incubation was carried out with a secondary antibody for 1 h at room temperature. After washing the membrane again, photographing and imaging were carried out on a gel imaging system by using ECL luminous liquid to expose a protein band. The antibody dilution ratios were as follows: IDO-1 primary antibody, 1:1000; GAPDH primary antibody, 1:1000; anti-rabbit secondary antibody, 1:5000.

### (3) Study results

As can be seen from the experimental results in FIG. 2, interferon-γ could effectively stimulate the expression of IDO-1 in Hela cells, and compound 1 could effectively reduce the expression of IDO-1 in Hela cells after co-incubation, indicating that compound 1 has a certain down-regulation effect on IDO protein levels in tumor cells, so as to achieve the role of regulating tumor microenvironment (TME). In the future, compound 1 can be used in combination with PD-1/PD-L1 antibody for immuno-oncology therapy, which has better clinical application potential.

Example 7: *In vivo* pharmacodynamic study of the compound of the present invention combined with PD-L1 on mouse B cell lymphoma A20 subcutaneous homograft tumor model

Test sample: compound 1 of the present invention, having a structure shown above.

Animals, cells, reagents & instruments:
A20 cells: mouse B cell lymphoma cells from ATCC.
Balb/c mice, 6- to 8-week-old, female, from Zhejiang Vital River Laboratory Animal Technology Co., Ltd.
PD-L1 antibody: InVivoMAb anti-mouse PD-L1 (B7-H1), clone number: 10F.9G2 (IgG2b), company: BioXcell, Cat. No. BE0101.
IgG control antibody: InVivoMAb rat IgG2b isotype control; clone number: LTF-2; company: BioXcell, Cat. No. BE0090.

### Test method:

### 1. Construction and grouping of tumor-bearing mice

A20 cells were cultured in monolayer *in vitro.* When the cells were in the exponential growth phase, the cells were harvested, counted, and inoculated.

0.04 mL of a cell suspension containing about 6 × 10⁵ A20 cells was inoculated subcutaneously into the back skin of female Balb/c mice. The mice were grouped for administration when the average volume of tumors reached approximately 60 mm³. Grouping method: Animals were weighed before administration and tumor volumes were measured. Grouping was designed in blocks according to the tumor volume, with 8 mice per group.

### 2. Administration regimen:

Administration was carried out as in Table 4 below.

**Table 4. Administration mode**

| Group | Dosage (mg/kg) | Number of animals | Route of administration | Frequency of administration | Administration period |
|---|---|---|---|---|---|
| Vehicle (0.5% MC) + IgG control antibody | 0 +10 | 8 | Oral intragastric administration + intraperitoneal injection | Qd +Q3d | 19 days |
| Compound 1 | 7.5 | 8 | Oral intragastric administration | Qd | 19 days |
| PD-L1 antibody | 10 | 8 | Intraperitoneal injection | Q3d | 19 days |
| Compound 1 + PD-L1 antibody | 7.5 +10 | 8 | Oral intragastric administration + intraperitoneal injection | Qd +Q3d | 19 days |

### 3. Experimental observation index

Animals were monitored daily for health status and mortality, body weight and tumor volume were measured twice a week, and samples were collected after the last administration. The therapeutic effect of tumor volume was evaluated by TGI%, and the evaluation was conducted as shown in Example 5.

### 4. Results as shown in Table 5:

**Table 5. Effect of compound 1 of the present invention combined with PD-L1 antibody on tumor growth of a mouse B cell lymphoma A20 subcutaneous homograft tumor model**

| Group | Number of animals | Tumor volume (mm³) | TGI (%) |
|---|---|---|---|
| Vehicle (0.5% MC) + IgG control antibody | 8 | 671 | -- |
| Compound 1 | 8 | 519 | 26 |
| PD-L1 antibody | 8 | 258 | 64 |
| Compound 1 + PD-L1 antibody | 8 | 165 | 76 |

As can be seen from the experimental results in Table 5, the combination of compound 1 with PD-L1 antibody had a significant inhibitory effect on the A20 cell homograft tumor model, and had an obvious effect of enhancing the efficacy by the combination, indicating that the clinical combination of the compound of the present invention with PD-L1 antibody has good clinical application potential for immuno-oncology therapy.

Example 8: *In vivo* pharmacodynamic study of the compound of the present invention combined with PD-L1 on mouse colon cancer MC38 subcutaneous homograft tumor model

Test sample: compound 1 of the present invention, having a structure shown above.

Animals, cells, reagents & instruments:
MC38 cells: mouse colon cancer cells from ATCC.
C57BL/6 mice, 6- to 8-week-old, female, from Shanghai Slac Laboratory Animal Co., Ltd.
PD-L1 antibody: InVivoMAb anti-mouse PD-L1 (B7-H1), clone number: 10F.9G2 (IgG2b), company: BioXcell, Cat. No. BE0101.
IgG control antibody: InVivoMAb rat IgG2b isotype control; clone number: LTF-2; company: BioXcell, Cat. No. BE0090.

### Test method:

### 1. Construction and grouping of tumor-bearing mice

MC38 cells were cultured in monolayer *in vitro.* When the cells were in the exponential growth phase, the cells were harvested, counted, and inoculated.

0.04 mL of a cell suspension containing about 3 × 10⁵ MC38 cells was inoculated subcutaneously into the back skin of female C57BL/6 mice. The mice were grouped for administration when the average volume of tumors reached approximately 60 mm³. Grouping method: Animals were weighed before administration and tumor volumes were measured. Grouping was designed in blocks according to the tumor volume, with 8 mice per group.

### 2. Administration regimen

Administration was carried out as in Table 6 below.

**Table 6. Administration mode**

| Group | Dosage (mg/kg) | Number of animals | Route of administration | Frequency of administration | Administration period |
|---|---|---|---|---|---|
| Vehicle (0.5% MC) + IgG control antibody | 0 +10 | 8 | Oral intragastric administration + intraperitoneal injection | Qd +Q3d | 25 days |
| Compound 1 | 7.5 | 8 | Oral intragastric administration | Qd | 25 days |
| PD-L1 antibody | 5 | 8 | Intraperitoneal injection | Q3d | 25 days |
| Compound 1 + PD-L1 antibody | 7.5 +5 | 8 | Oral intragastric administration + intraperitoneal injection | Qd +Q3d | 25 days |

### 3. Experimental observation index

Animals were monitored daily for health status and mortality, body weight and tumor volume were measured twice a week, and samples were collected after the last administration. The therapeutic effect of tumor volume was evaluated by TGI%, and the evaluation was conducted as shown in Example 5.

### 4. Results as shown in Table 7:

**Table 7. Effect of compound 1 of the present invention combined with PD-L1 antibody on tumor growth of a mouse colon cancer MC38 subcutaneous homograft tumor model**

| Group | Number of animals | Tumor volume (mm³) | TGI (%) |
|---|---|---|---|
| Vehicle (0.5% MC) | 8 | 1036 | -- |
| + IgG control antibody | | | |
| Compound 1 | 8 | 554 | 50 |
| PD-L1 antibody | 8 | 314 | 70 |
| Compound 1 + PD-L1 antibody | 8 | 146 | 87 |

As can be seen from the experimental results in Table 7, the combination of compound 1 with PD-L1 antibody had a significant inhibitory effect on the MC38 cell homograft tumor model, and had an obvious effect of enhancing the efficacy by the combination, indicating that the clinical combination of the compound of the present invention with PD-L1 antibody has good clinical application potential for immuno-oncology therapy.

Example 9: *In vivo* pharmacodynamic study of the compound of the present invention combined with PD-L1 on mouse pancreatic cancer Pan02 subcutaneous homograft tumor model

Test sample: compound 1 of the present invention, having a structure shown above.

Animals, cells, reagents & instruments:
Pan02 cells: mouse pancreatic cancer cells from ATCC.
C57BL/6 mice, 6- to 8-week-old, female, from Shanghai Slac Laboratory Animal Co., Ltd.
PD-L1 antibody: InVivoMAb anti-mouse PD-L1 (B7-H1), clone number: 10F.9G2 (IgG2b), company: BioXcell, Cat. No. BE0101
IgG control antibody: InVivoMAb rat IgG2b isotype control, clone number: LTF-2; company: BioXcell, Cat. No. BE0090

### Test method:

### 1. Construction and grouping of tumor-bearing mice

Pan02 cells were cultured in monolayer *in vitro.* When the cells were in the exponential growth phase, the cells were harvested, counted, and inoculated.

0.06 mL of a cell suspension containing about 1 × 10⁶ Pan02 cells was inoculated subcutaneously into the back skin of female C57BL/6 mice. The mice were grouped for administration when the average volume of tumors reached approximately 60 mm³. Grouping method: Animals were weighed before administration and tumor volumes were measured. Grouping was designed in blocks according to the tumor volume, with 7 mice per group.

### 2. Administration regimen

Administration was carried out as in Table 8 below.

**Table 8. Administration mode**

| Group | Dosage (mg/kg) | Number of animals | Route of administration | Frequency of administration | Administration period |
|---|---|---|---|---|---|
| Vehicle (0.5% MC) + IgG control antibody | 0 +10 | 7 | Oral intragastric administration + intraperitoneal injection | Qd +Q3d | 25 days |
| Compound 1 | 7.5 | 7 | Oral intragastric administration | Qd | 25 days |
| PD-L1 antibody | 10 | 7 | Intraperitoneal injection | Q3d | 25 days |
| Compound 1 + PD-L1 antibody | 7.5 +10 | 7 | Oral intragastric administration + intraperitoneal injection | Qd +Q3d | 25 days |

### 3. Experimental observation index

Animals were monitored daily for health status and mortality, body weight and tumor volume were measured twice a week, and samples were collected after the last administration. The therapeutic effect of tumor volume was evaluated by TGI%, and the evaluation was conducted as shown in Example 5.

### 4. Results as shown in Table 9:

**Table 9. Effect of the compound 1 of the present invention combined with PD-L1 antibody on tumor growth of a mouse pancreatic cancer Pan02 subcutaneous homograft tumor model**

| Group | Number of animals | Tumor volume (mm³) | TGI (%) |
|---|---|---|---|
| Vehicle (0.5% MC) + IgG control antibody | 4^{#} | 1595 | -- |
| Compound 1 | 7 | 921 | 71 |
| PD-L1 antibody | 4^{#} | 1889 | -6 |
| Compound 1 + PD-L1 antibody | 7 | 501 | 81 |

# represents that three out of seven experimental mice were terminated due to tumor rupture or impaired quality of survival of the mice, and finally four mice were used for data analysis.

As can be seen from the experimental results in Table 9, the combination of compound 1 with PD-L1 antibody had a significant inhibitory effect on the Pan02 cell homograft tumor model, and had an obvious effect of enhancing the efficacy by the combination. Meanwhile, as observed in the experiment, no tumor rupture or impaired quality of survival of the mice was found in the group of compound 1 + PD-L1 antibody, and the quality of survival of the mice was improved, indicating that the clinical combination of the compound of the present invention with PD-L1 antibody has good clinical application potential for immuno-oncology therapy.

## Claims

1. A pharmaceutical composition of a multi-kinase inhibitor compound shown in formula (I) or a pharmaceutically acceptable salt, a stereoisomer or a crystal form thereof and a programmed cell death protein 1/programmed cell death protein 1 ligand 1 (PD-1/PD-L1) antibody, 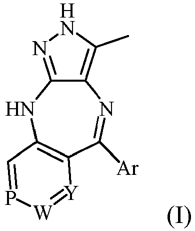 wherein,
Ar is selected from phenyl and 5-6 membered heteroaryl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, oxa-C₅₋₈ cycloalkyloxy, halogenated C₁₋₄ alkoxy, C₃₋₆ cycloalkylamino, C₁₋₄ alkylsulfonyl, C₃₋₆ cycloalkylsulfonyl, C₁₋₄ alkylcarbonyl, C₃₋₆ cycloalkylcarbonyl, -(CH₂)ₙ-C₃₋₁₀ cycloalkyl, -(CH₂)ₙ-(5-11) membered heterocyclyl, and - (CH₂)ₙ-(5-10) membered heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

2. A kit for treating a cancer, comprising (a) a multi-kinase inhibitor compound shown in formula (I) or a pharmaceutically acceptable salt, a stereoisomer or a crystal form thereof, (b) a programmed cell death protein 1/programmed cell death protein 1 ligand 1 (PD-1/PD-L1) antibody, and (c) a package insert, 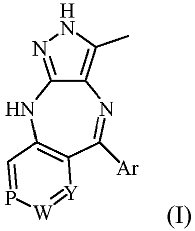 wherein,
Ar is selected from phenyl and 5-6 membered heteroaryl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, oxa-C₅₋₈ cycloalkyloxy, halogenated C₁₋₄ alkoxy, C₃₋₆ cycloalkylamino, C₁₋₄ alkylsulfonyl, C₃₋₆ cycloalkylsulfonyl, C₁₋₄ alkylcarbonyl, C₃₋₆ cycloalkylcarbonyl, -(CH₂)ₙ-C₃₋₁₀ cycloalkyl, -(CH₂)ₙ-(5-11) membered heterocyclyl, and - (CH₂)ₙ-(5-10) membered heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

3. The pharmaceutical composition according to claim 1 or the kit according to claim 2, wherein in the compound of formula (I):
Ar is selected from phenyl, Ar can be optionally substituted with 1 to 3 R₆, and each R₆ is independently selected from hydrogen, amino, cyano, halogen, C₁₋₄ alkyl, trifluoromethyl, and methanesulfonyl;
Y is selected from CR₃; P is selected from CR₄; W is selected from N;
R₃ is selected from hydrogen and C₁₋₄ alkyl;
R₄ is selected from hydrogen, hydroxyl, amino, carboxyl, cyano, nitro, halogen, C₁₋₄ alkoxy, C₃₋₆ cycloalkyloxy, halogenated C₁₋₄ alkoxy, C₃₋₆ cycloalkylamino, C₁₋₄ alkylsulfonyl, C₁₋₄ alkylcarbonyl, C₃₋₆ cycloalkyl-carbonyl, -(CH₂)ₙ-C₃₋₆ cycloalkyl, -(CH₂)ₙ-(5-6) membered monocyclic heterocyclyl, -(CH₂)ₙ-(7-11) membered fused heterocyclyl, -(CH₂)ₙ-(5-6) membered monocyclic heteroaryl, and -(CH₂)ₙ-(8-10) membered fused heteroaryl, wherein n = 0-6, a ring-forming S atom in the cycloalkyl, heterocyclyl, and heteroaryl can be optionally oxidized to S(O) or S(O)₂, a ring-forming C atom in the cycloalkyl, heterocyclyl, and heteroaryl can be optionally oxidized to C(O), and the cycloalkyl, heteroaryl, and heterocyclyl can be optionally substituted with one or more substituents independently selected from C₁₋₃ alkyl and C₃₋₆ cycloalkyl.

4. The pharmaceutical composition or the kit according to claim 3, wherein formula (I) is selected from compounds with the following structures: 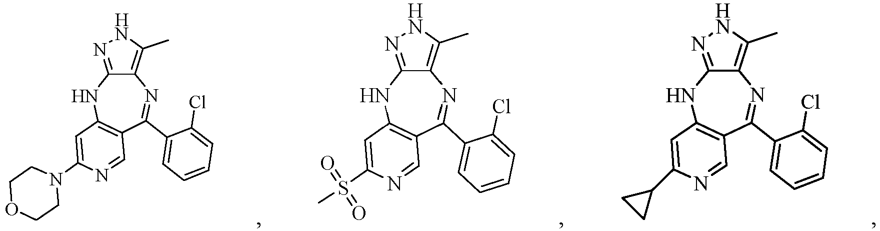 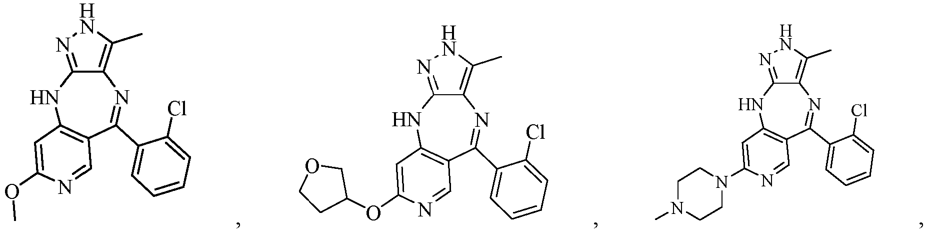 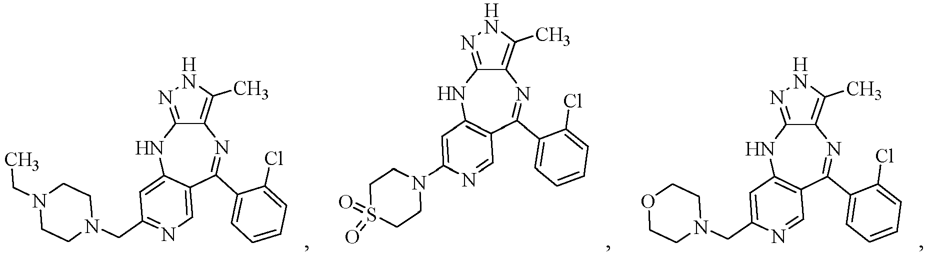 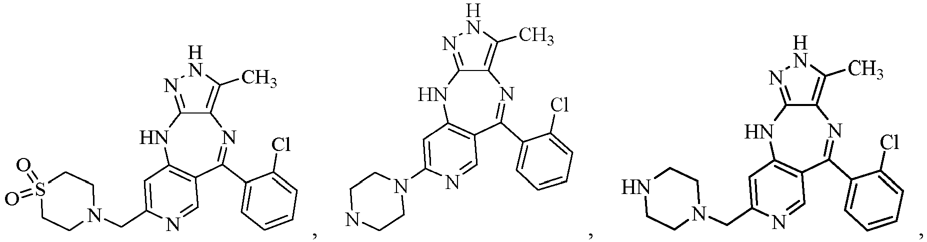 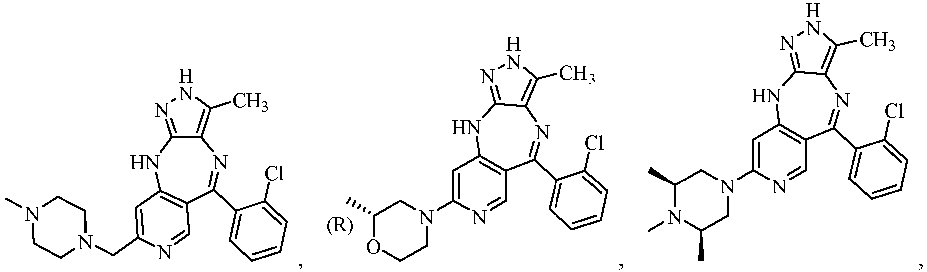 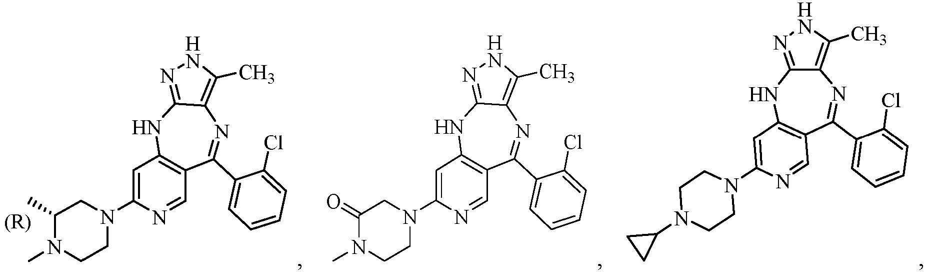 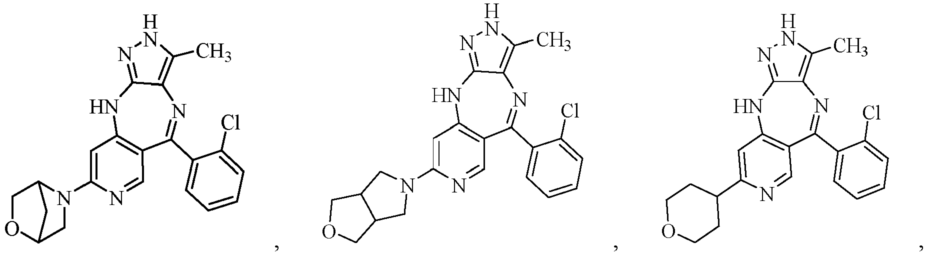 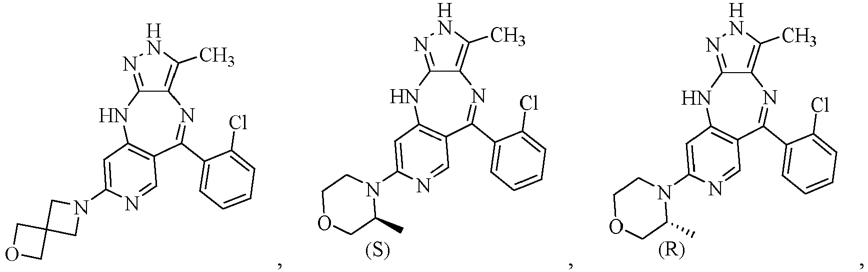 and

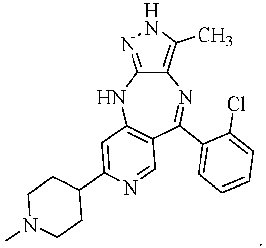

5. The pharmaceutical composition or the kit according to claim 4, wherein formula (I) is selected from a compound with the following structure:

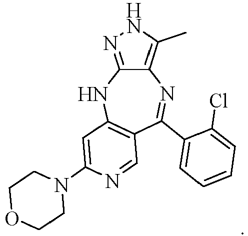

6. The pharmaceutical composition according to claim 1 or the kit according to claim 2, wherein the programmed cell death protein 1/programmed cell death protein 1 ligand 1 (PD-1/PD-L1) antibody is a monoclonal antibody.

7. The pharmaceutical composition according to claim 1 or the kit according to claim 2, wherein the pharmaceutical composition or the kit can further comprise an additional anti-cancer agent or immunosuppressant.

8. Use of the pharmaceutical composition according to claim 1 or the kit according to claim 2 in the manufacture of a medicament for preventing and/or treating a cancer mediated by multi-kinase abnormalities, wherein the cancer comprises lung cancer, squamous cell carcinoma, bladder cancer, gastric cancer, ovarian cancer, peritoneal cancer, breast cancer, head and neck cancer, endometrial cancer, corpus uteri cancer, rectal cancer, liver cancer, cholangiocarcinoma, gallbladder carcinoma, pancreatic cancer, renal cancer, renal pelvis cancer, esophageal cancer, esophageal adenocarcinoma, glioma, thyroid cancer, female reproductive system cancer, neurofibromatosis, bone cancer, skin cancer, brain cancer, colon cancer, testicular cancer, oral cancer, pharyngeal cancer, hematological cancer, villous adenoma of large intestine, melanoma, cytoma, and sarcoma.

9. The use according to claim 8, wherein the multi-kinase inhibitor compound shown in formula (I) and the programmed cell death protein 1/programmed cell death protein 1 ligand 1 (PD-1/PD-L1) antibody can be used in combination in a single formulation or administered to a patient as independent agents simultaneously, sequentially, or intermittently.

## Patentansprüche

1. Eine pharmazeutische Zusammensetzung einer Multi-Kinase Inhibitorverbindung, wie dargestellt in Formel I, oder ein pharmazeutisch akzeptables Salz, ein Stereoisomer oder eine Kristallform davon, und ein programmierter Zelltodprotein-1 / programmierter Zelltodprotein-1-Ligand-1 (PD-1/PD-L1) Antikörper 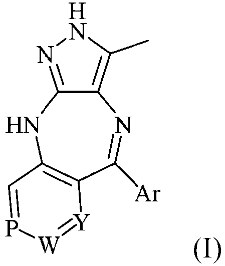 wobei,
Ar ausgewählt ist aus Phenyl und 5-6-gliedrigem Heteroaryl, Ar optional mit 1 bis 3 R₆ substituiert sein kann, und jedes R₆ unabhängig ausgewählt ist aus Wasserstoff, Amino, Cyano, Halogen, C₁₋₄-Alkyl, Trifluormethyl und Methansulfonyl;
Y ausgewählt ist aus CR₃; P ausgewählt ist aus CR₄; W ausgewählt ist aus N;
R₃ ausgewählt ist aus Wasserstoff und C₁₋₄-Alkyl;
R₄ ausgewählt ist aus Wasserstoff, Hydroxyl, Amino, Carboxyl, Cyano, Nitro, Halogen, C₁₋₄-Alkoxy, C₃₋₆-Cycloalkyloxy, Oxa-C₅₋₈-Cycloalkyloxy, halogeniertem C₁₋₄-Alkoxy, C₃₋₆ Cycloalkylamino, C₁₋₄-Alkylsulfonyl, C₃₋₆-Cycloalkylsulfonyl, C₁₋₄-Alkylcarbonyl, C₃₋₆-Cycloalkylcarbonyl, -(CH₂)ₙ-C₃₋₁₀-Cycloalkyl, -(CH₂)ₙ-(5-11)-gliedrigem Heterocyclyl und -(CH₂)ₙ-(5-10)-gliedrigem Heteroaryl, wobei n = 0-6, einem ringbildenden S-Atom im Cycloalkyl, Heterocyclyl und Heteroaryl optional zu S(O) oder S(O)₂ oxidiert werden kann, einem ringbildenden C-Atom im Cycloalkyl, Heteroaryl und Heteroaryl optional zu C(O) oxidiert werden kann, und das Cycloalkyl, Heteroaryl und Heteroaryl optional mit einem oder mehreren Substituenten substituiert sein können, die unabhängig ausgewählt sind aus C₁₋₃-Alkyl und C₃₋₆-Cycloalkyl.

2. Ein Kit zum Behandeln von Krebs, umfassend (a) eine Multi-Kinase-Inhibitorverbindung, wie dargestellt in der Formel (I), oder ein pharmazeutisch akzeptables Salz, ein Stereoisomer oder eine Kristallform davon,
(b) einen programmierten Zelltodprotein-1 / programmierten Zelltodprotein-1-Ligand-1 (PD-1/PDL1) Antikörper und (c) eine Packungsbeilage, 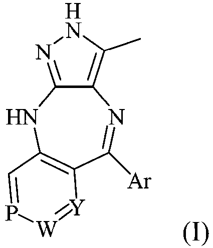 wobei,
Ar ausgewählt ist aus Phenyl und 5-6-gliedrigem Heteroaryl, Ar optional mit 1 bis 3 R₆ substituiert sein kann und jedes R₆ unabhängig ausgewählt ist aus Wasserstoff, Amino, Cyano, Halogen, C1-4-Alkyl, Rifluormethyl und Methansulfonyl;
Y ausgewählt ist aus CR₃; P ausgewählt ist aus CR₄; W ausgewählt ist aus N; R₃ ausgewählt ist aus Wasserstoff und C₁₋₄-Alkyl; R₄ ausgewählt ist aus Wasserstoff, Hydroxyl, Amino, Carboxyl, Cyano, Nitro, Halogen, C₁₋₄-Alkoxy, C₃₋₆-Cycloalkyloxy, Oxa-C₅₋₈-Cycloalkyloxy, halogeniertem C₁₋₄-Alkoxy, C₃₋₆-Cycloalkylamino, C₁₋₄-Alkylsulfonyl, C₃₋₆-Cycloalkylsulfonyl, C₁₋₄-Alkylcarbonyl, C₃₋₆-Cycloalkylcarbonyl, - (CH₂)ₙ-C₃₋₁₀-Cycloalkyl, -(CH₂)ₙ-(5-11)-gliedrigem Heterocyclyl und -(CH₂)ₙ-(5-10)-gliedrigem Heteroaryl, wobei n = 0-6, einem ringbildenden S-Atom in dem Cycloalkyl, Heterocyclyl und Heteroaryl optional zu S(O) oder S(O)₂ oxidiert werden kann, einem ringbildenden C-Atom in dem Cycloalkyl, Heteroaryl optional zu C(O) oxidiert sein kann, und das Cycloalkyl, Heteroaryl und Heterocyclyl optional mit einem oder mehreren Substituenten substituiert sein können, die unabhängig ausgewählt sind aus C₁₋₃-Alkyl und C₃₋₆-Cycloalkyl.

3. Die pharmazeutische Zusammensetzung gemäß Anspruch 1 oder das Kit gemäß Anspruch 2, wobei in der Verbindung der Formel (I):
Ar aus Phenyl ausgewählt ist, Ar optional mit 1 bis 3 R₆ substituiert sein kann und jedes R₆ unabhängig aus Wasserstoff, Amino, Cyano, Halogen, C₁₋₄-Alkyl, Trifluormethyl und Methansulfonyl ausgewählt ist;
Y ausgewählt ist aus CR₃; P ausgewählt ist aus CR₄; W ausgewählt ist aus N;
R₃ ausgewählt ist aus Wasserstoff und C₁₋₄-Alkyl; R₄ ausgewählt ist aus Wasserstoff, Hydroxyl, Amino, Carboxyl, Cyano, Nitro, Halogen, C₁₋₄-Alkoxy, C₃₋₆-Cycloalkyloxy, halogeniertem C₁₋₄-Alkoxy, C₃₋₆-Cycloalkylamino, C₁₋₄-Alkylsulfonyl, C₁₋₄-Alkylcarbonyl, C₃₋₆-Cycloal kylcarbonyl, -(CH₂)ₙ-C₃₋₆-Cycloalkyl, -(CH₂)ₙ-(5-6)-gliedrigem monocyclischem Heterocyclyl, -(CH₂)ₙ-(7-11)-gliedrigem kondensiertem Heterocyclyl, - (CH₂)ₙ-(5-6)-gliedrigem monocyclischem Heteroaryl und -(CH₂)ₙ-(8-10)-gliedrigem kondensiertem Heteroaryl, wobei n = 0-6, einem ringbildenden S-Atom im Cycloalkyl, Heterocyclyl und Heteroaryl optional zu S(O) oder S(O)₂ oxidiert werden kann, einem ringbildenden C-Atom im Cycloalkyl, Heterocyclyl und Heteroaryl optional zu C(O) oxidiert sein kann, und das Cycloalkyl, Heteroaryl und Heterocyclyl optional mit einem oder mehreren Substituenten substituiert sein können, die unabhängig voneinander aus C₁₋₃-Alkyl und C₃₋₆-Cycloalkyl ausgewählt sind.

4. Die pharmazeutische Zusammensetzung oder das Kit gemäß Anspruch 3, wobei die Formel (I) aus Verbindungen mit den folgenden Strukturen ausgewählt ist: 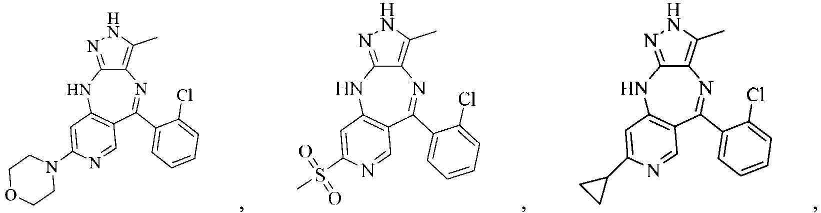 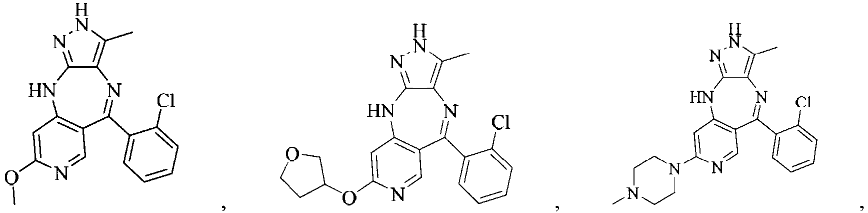 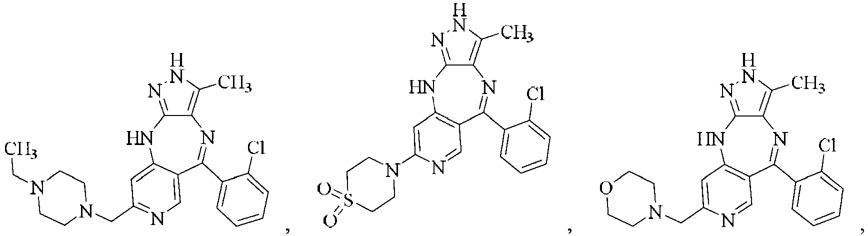 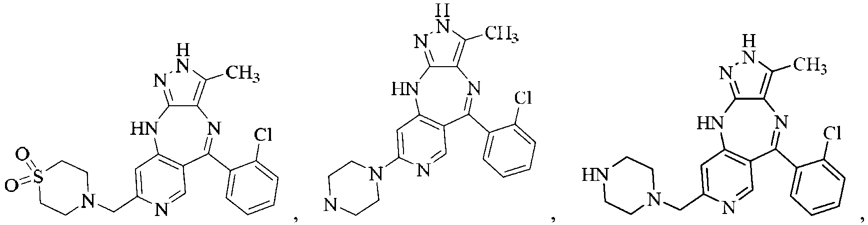 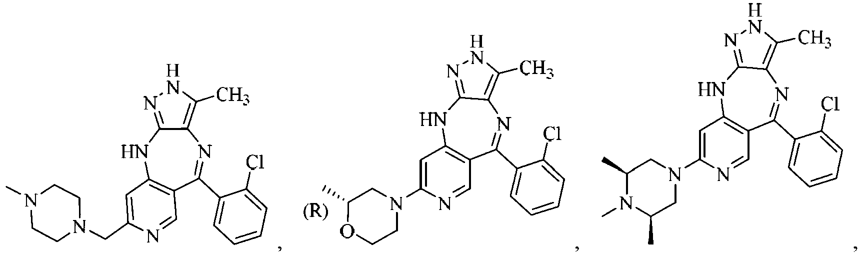 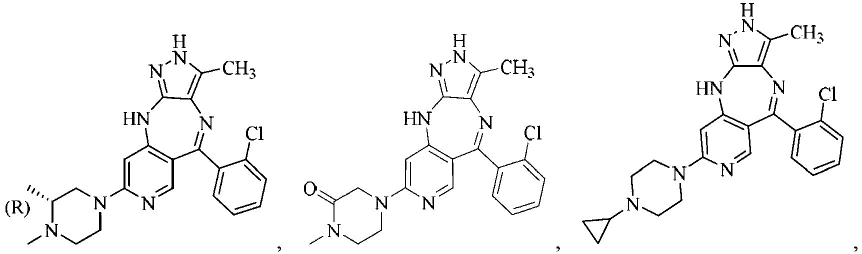 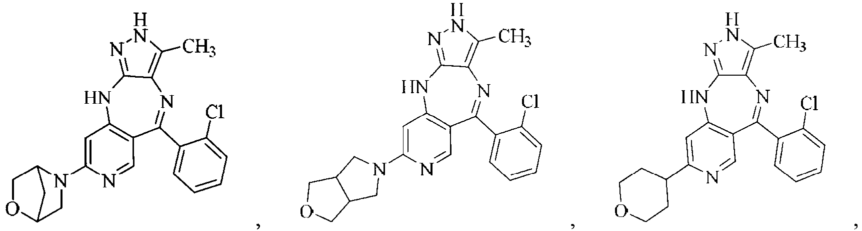 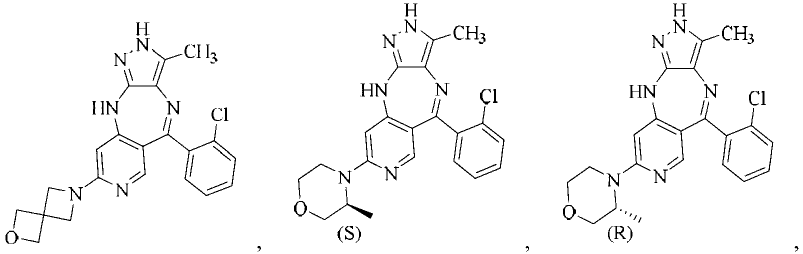 und

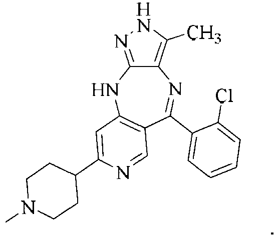

5. Die pharmazeutische Zusammensetzung oder das Kit gemäß Anspruch 4, wobei die Formel (I) aus einer Verbindung mit der folgenden Struktur ausgewählt ist:

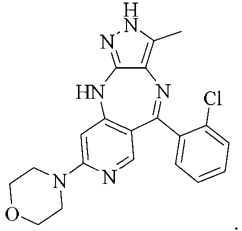

6. Die pharmazeutische Zusammensetzung gemäß Anspruch 1 oder das Kit gemäß Anspruch 2, wobei der programmierte Zelltodprotein-1 / programmierte Zelltodprotein-1-Ligand-1 (PD-1/PD-L1) Antikörper ein monoklonaler Antikörper ist.

7. Die pharmazeutische Zusammensetzung gemäß Anspruch 1 oder das Kit gemäß Anspruch 2, wobei die pharmazeutische Zusammensetzung oder das Kit zusätzlich ein weiteres Antikrebsmittel oder Immunsuppressivum enthalten kann.

8. Verwendung der pharmazeutischen Zusammensetzung gemäß Anspruch 1 oder des Kits gemäß Anspruch 2 zur Herstellung eines Medikaments zum Vorbeugen und/oder Behandeln von Krebs, der durch Multi-Kinase-Anomalien vermittelt wird, wobei der Krebs Lungenkrebs, Plattenepithelkarzinom, Blasenkrebs, Magenkrebs, Eierstockkrebs, Peritonealkrebs, Brustkrebs, Kopf- und Halskrebs, Endometriumkarzinom, Gebärmutterkörperkrebs, Rektumkarzinom, Leberkrebs, Cholangiokarzinom, Gallenblasenkarzinom, Bauchspeicheldrüsenkrebs, Nierenkrebs, Nierenbeckenkrebs, Speiseröhrenkrebs, Speiseröhrenadenokarzinom, Gliom, Schilddrüsenkrebs, Krebs des weiblichen Fortpflanzungssystems, Neurofibromatose, Knochenkrebs, Hautkrebs, Hirntumor, Dickdarmkrebs, Hodenkrebs, Mundhöhlenkrebs, Rachenkrebs, hämatologischer Krebs, villöses Adenom des Dickdarms, Melanom, Zytom und Sarkom umfasst.

9. Verwendung gemäß Anspruch 8, wobei die in Formel (I) dargestellte Multi-Kinase-Inhibitorverbindung und der programmierte Zelltod-Protein-1 / programmierte ZelltodProtein-1-Ligand-1 (PD-1/PD-L1) Antikörper in Kombination in einer einzigen Formulierung verwendet oder einem Patienten als unabhängige Wirkstoffe gleichzeitig, nacheinander oder intermittierend verabreicht werden können.

## Revendications

1. Composition pharmaceutique d'un composé inhibiteur multi-kinase présenté dans la formule (I) ou son sel, stéréoisomère ou sa forme cristalline pharmaceutiquement acceptable et un anticorps de protéine de mort cellulaire programmée 1/ligand 1 de protéine de mort cellulaire programmée 1 (PD-1/PD-L1), 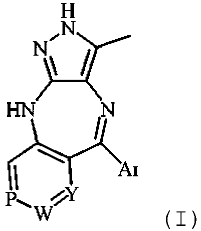 dans laquelle,
Ar est sélectionné parmi un groupe phényle et hétéroaryle à 5 à 6 chaînons, Ar peut être éventuellement substitué par 1 à 3 R₆, et chaque R₆ est indépendamment sélectionné parmi un groupe hydrogéno, amino, cyano, halogéno, alkyle en C₁ à C₄, trifluorométhyle, et méthanesulfonyle ;
Y est sélectionné parmi CR₃ ; P est sélectionné parmi CR₄ ; W est sélectionné parmi N ;
R₃ est sélectionné parmi un groupe hydrogéno et alkyle en C₁ à C₄ ;
R₄ est sélectionné parmi un groupe hydrogéno, hydroxyle, amino, carboxyle, cyano, nitro, halogéno, alcoxy en C₁ à C₄, cycloalkyloxy en C₃ à C₆, oxa-(cycloalkyloxy en C₅ à C₈), alcoxy en C₁ à C₄ halogéné, cycloalkylamino en C₃ à C₆, alkylsulfonyle en C₁ à C₄, cycloalkylsulfonyle en C₃ à C₆, alkylcarbonyle en C₁ à C₄, cycloalkylcarbonyle en C₃ à C₆, -(CH₂)ₙ- (cycloalkyle en C₃ à C₁₀), -(CH₂)ₙ- (hétérocyclyle à 5 à 11 chaînons), et - (CH₂)ₙ-(hétéroaryle à 5 à 10 chaînons), dans laquelle n = 0 à 6, un atome S formant un cycle dans les groupes cycloalkyle, hétérocyclyle, et hétéroaryle peuvent être éventuellement oxydés en S(O) ou S(O)₂, un atome C formant un cycle dans les groupes cycloalkyle, hétérocyclyle, et hétéroaryle peuvent être éventuellement oxydés en C(O), et les groupes cycloalkyle, hétéroaryle, et hétérocyclyle peuvent être éventuellement substitués par un ou plusieurs substituants indépendamment sélectionnés parmi un groupe alkyle en C₁ à C₃ et cycloalkyle en C₃ à C₆.

2. Kit de traitement d'un cancer, comprenant (a) un composé inhibiteur multi-kinase présenté dans la formule (I) ou son sel, stéréoisomère ou sa forme cristalline pharmaceutiquement acceptable, (b) un anticorps de protéine de mort cellulaire programmée 1/ligand 1 de protéine de mort cellulaire programmée 1 (PD-1/PD-L1), et (c) une étiquette d'emballage, 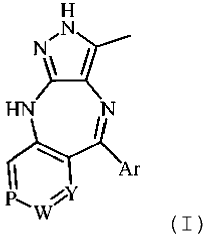 dans laquelle,
Ar est sélectionné parmi un groupe phényle et hétéroaryle à 5 à 6 chaînons, Ar peut être éventuellement substitué par 1 à 3 R₆, et chaque R₆ est indépendamment sélectionné parmi un groupe hydrogéno, amino, cyano, halogéno, alkyle en C₁ à C₄, trifluorométhyle, et méthanesulfonyle ;
Y est sélectionné parmi CR₃ ; P est sélectionné parmi CR₄ ; W est sélectionné parmi N ;
R₃ est sélectionné parmi un groupe hydrogéno et alkyle en C₁ à C₄ ;
R₄ est sélectionné parmi un groupe hydrogéno, hydroxyle, amino, carboxyle, cyano, nitro, halogéno, alcoxy en C₁ à C₄, cycloalkyloxy en C₃ à C₆, oxa-(cycloalkyloxy en C₅ à C₈), alcoxy en C₁ à C₄ halogéné, cycloalkylamino en C₃ à C₆, alkylsulfonyle en C₁ à C₄, cycloalkylsulfonyle en C₃ à C₆, alkylcarbonyle en C₁ à C₄, cycloalkylcarbonyle en C₃ à C₆, -(CH₂)ₙ-(cycloalkyle en C₃ à C₁₀), -(CH₂)ₙ-(hétérocyclyle à 5 à 11 chaînons), et - (CH₂)ₙ-(hétéroaryle à 5 à 10 chaînons), dans laquelle n = 0 à 6, un atome S formant un cycle dans les groupes cycloalkyle, hétérocyclyle, et hétéroaryle peuvent être éventuellement oxydés en S(O) ou S(O)₂, un atome C formant un cycle dans les groupes cycloalkyle, hétérocyclyle, et hétéroaryle peuvent être éventuellement oxydés en C(O), et les groupes cycloalkyle, hétéroaryle, et hétérocyclyle peuvent être éventuellement substitués par un ou plusieurs substituants indépendamment sélectionnés parmi un groupe alkyle en C₁ à C₃ et cycloalkyle en C₃ à C₆.

3. Composition pharmaceutique selon la revendication 1 ou kit selon la revendication 2, dans lequel·laquelle dans le composé de formule (I) :
Ar est sélectionné parmi un groupe phényle, Ar peut être éventuellement substitué par 1 à 3 R₆, et chaque R₆ est indépendamment sélectionné parmi un groupe hydrogéno, amino, cyano, halogéno, alkyle en C₁ à C₄, trifluorométhyle, et méthanesulfonyle ;
Y est sélectionné parmi CR₃ ; P est sélectionné parmi CR₄ ; W est sélectionné parmi N ;
R₃ est sélectionné parmi un groupe hydrogéno et alkyle en C₁ à C₄ ;
R₄ est sélectionné parmi un groupe hydrogéno, hydroxyle, amino, carboxyle, cyano, nitro, halogéno, alcoxy en C₁ à C₄, cycloalkyloxy en C₃ à C₆, alcoxy en C₁ à C₄ halogéné, cycloalkylamino en C₃ à C₆, alkylsulfonyle en C₁ à C₄, alkylcarbonyle en C₁ à C₄, (cycloalkyle en C₃ à C₆) -carbonyle, -(CH₂)ₙ- (cycloalkyle en C₃ à C₆), -(CH₂)ₙ-(hétérocyclyle monocyclique à 5 à 6 chaînons), -(CH₂)ₙ-(hétérocyclyle à 7 à 11 chaînons fusionnés), -(CH₂)ₙ-(hétéroaryle monocyclique à 5 à 6 chaînons), et -(CH₂)ₙ-(hétéroaryle à 8 à 10 chaînons fusionnés), dans laquelle n = 0 à 6, un atome S formant un cycle dans les groupes cycloalkyle, hétérocyclyle, et hétéroaryle peuvent être éventuellement oxydés en S(O) ou S(O)₂, un atome C formant un cycle dans les groupes cycloalkyle, hétérocyclyle, et hétéroaryle peuvent être éventuellement oxydés en C(O), et les groupes cycloalkyle, hétéroaryle, et hétérocyclyle peuvent être éventuellement substitués par un ou plusieurs substituants indépendamment sélectionnés parmi un groupe alkyle en C₁ à C₃ et cycloalkyle en C₃ à C₆.

4. Composition pharmaceutique ou kit selon la revendication 3, dans lequel·laquelle la formule (I) est sélectionnée parmi les composés des structures suivantes : 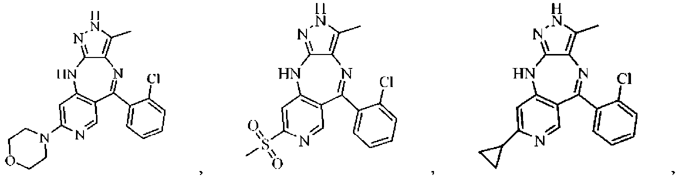 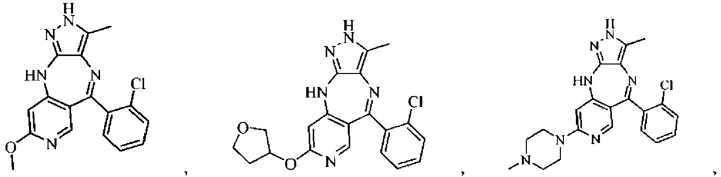 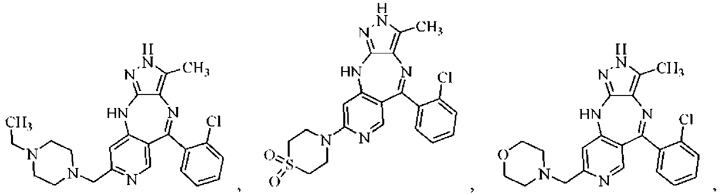 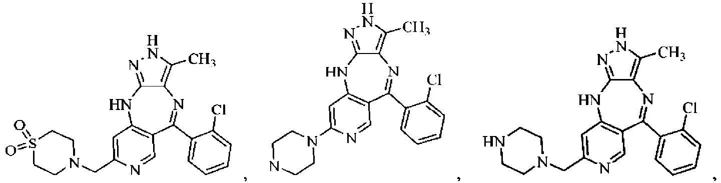 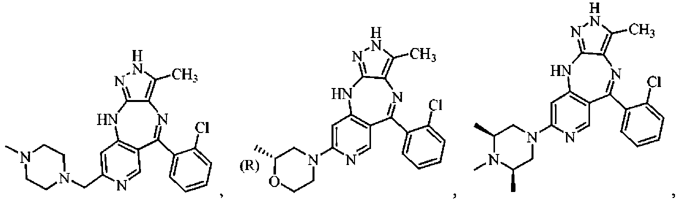 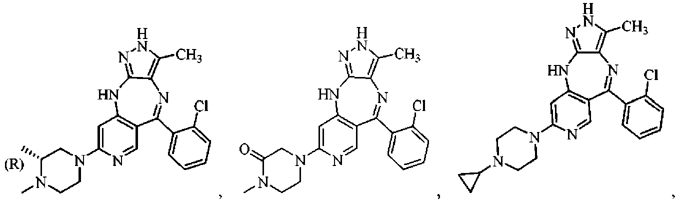 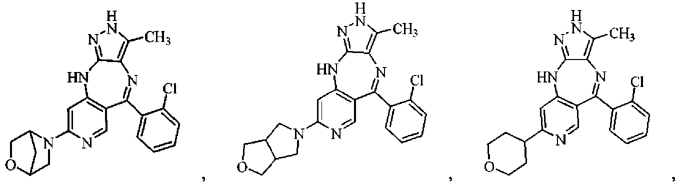 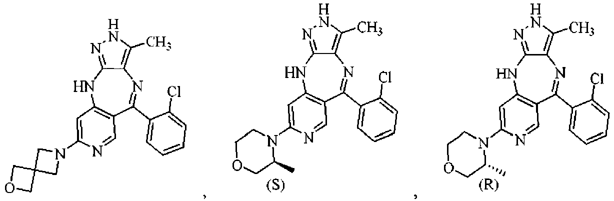 et

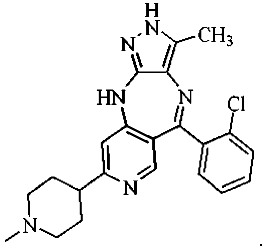

5. Composition pharmaceutique ou kit selon la revendication 4, dans lequel·laquelle la formule (I)est sélectionnée parmi un composé ayant la structure suivante :

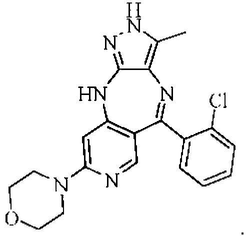

6. Composition pharmaceutique selon la revendication 1 ou kit selon la revendication 2, dans lequel·laquelle l'anticorps de protéine de mort cellulaire programmée 1/ligand 1 de protéine de mort cellulaire programmée 1 (PD-1/PD-L1) est un anticorps monoclonal.

7. Composition pharmaceutique selon la revendication 1 ou kit selon la revendication 2, dans lequel·laquelle la composition pharmaceutique ou le kit peut en outre comprendre un agent anticancéreux additionnel ou un agent immunosuppresseur.

8. Utilisation de la composition pharmaceutique selon la revendication 1 ou du kit selon la revendication 2 dans la fabrication d'un médicament pour prévenir et/ou traiter un cancer à médiation par des anomalies multi-kinases, dans laquelle le cancer comprend le cancer du poumon, le carcinome des cellules squameuses, le cancer de la vessie, le cancer gastrique, le cancer de l'ovaire, le cancer du péritoine, le cancer du sein, le cancer de la tête et du cou, le cancer de l'endomètre, le cancer du corps utérin, le cancer rectal, le cancer du foie, le cholangiocarcinome, le carcinome de la vésicule biliaire, le cancer pancréatique, le cancer rénal, le cancer du pelvis rénal, le cancer de l'œsophage, l'adénocarcinome de l'œsophage, le gliome, le cancer de la thyroïde, le cancer du système reproducteur chez la femme, la neurofibromatose, le cancer des os, le cancer de la peau, le cancer du cerveau, le cancer du côlon, le cancer des testicules, le cancer de la bouche, le cancer pharyngé, le cancer hématologique, l'adénome villeux du gros intestin, le mélanome, le cytome, et le sarcome.

9. Utilisation selon la revendication 8, dans laquelle le composé inhibiteur multi-kinase présenté dans la formule (I) et l'anticorps de protéine de mort cellulaire programmée 1/ligand 1 de protéine de mort cellulaire programmée 1 (PD-1/PD-L1) peuvent être utilisés en combinaison dans une formulation unique ou administrés chez un patient comme agents indépendants simultanément, de manière séquentielle, ou de manière intermittente.
